(19) Europäisches Patentamt
European Patent Office
Office européen des brevets

(11) EP 1 472 221 B1

(12) # EUROPÄISCHE PATENTSCHRIFT

(45) Veröffentlichungstag und Bekanntmachung des Hinweises auf die Patenterteilung:
**22.03.2006 Patentblatt 2006/12**

(21) Anmeldenummer: **02781281.7**

(22) Anmeldetag: **23.10.2002**

(51) Int Cl.:
*C07D 209/42* (2006.01) *C07D 401/06* (2006.01)
*C07D 403/12* (2006.01) *C07D 403/06* (2006.01)
*A61K 31/40* (2006.01)

(86) Internationale Anmeldenummer:
**PCT/EP2002/011831**

(87) Internationale Veröffentlichungsnummer:
**WO 2003/037863 (08.05.2003 Gazette 2003/19)**

(54) **SUBSTITUIERTE INDOLE VERFAHREN ZU DEREN HERSTELLUNG UND DEREN VERWENDUNG ZUR SCHMERZBEKÄMFUNG**

SUBSTITUTED INDOLES, METHOD FOR PRODUCTION AND USE THEREOF FOR THE INHIBITION OF PAIN

INDOLES SUBSTITUES, PROCEDE POUR LEUR PREPARATION ET LEUR UTILISATION POUR LUTTER CONTRE LA DOULEUR

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **29.10.2001 DE 10153346**

(43) Veröffentlichungstag der Anmeldung:
**03.11.2004 Patentblatt 2004/45**

(73) Patentinhaber: **Grünenthal GmbH**
**52078 Aachen (DE)**

(72) Erfinder:
- **SATTLEGGER, Michael, Dr.**
  **53123 Bonn (DE)**
- **BUSCHMANN, Helmut, Dr.**
  **E-08950 Esplugues de Llobregat (ES)**
- **PRZEWOSNY, Michael, Dr.**
  **52062 Aachen (DE)**
- **ENLGBERGER, Werner, Dr.**
  **52223 Stolberg (DE)**
- **KOEGEL, Babette-Yvonne, Dr.**
  **52379 Langerwehe-Hamich (DE)**
- **SCHICK, Hans, Prof., Dr.**
  **10405 Berlin (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:
**EP-A- 0 853 085** **WO-A-01/47882**
**WO-A-01/47885** **WO-A-97/23216**
**CH-A- 635 584** **GB-A- 925 429**
**GB-A- 1 501 475** **US-A- 4 113 866**
**US-A- 5 145 845**

- **K. FLICK ET AL.: "Untersuchungen zur Chemischen struktur and analgetischen Wirkung von Phenylsubstituierten Aminomethylcyclohexanolen" ARZNEIMITTEL FORSCHUNG, Bd. 28, Nr. 1a, 1978, Seiten 107-113, XP000608150**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft substituierte Indole, Verfahren zu deren Herstellung, Arzneimittel enthaltend diese Verbindungen sowie die Verwendung dieser Verbindungen zur Herstellung von Arzneimitteln.

Die Behandlung von Schmerz hat in der Medizin große Bedeutung. Es besteht ein weltweiter Bedarf an wirksamen Schmerztherapien. Der dringende Handlungsbedarf für eine patientengerechte und zielortentierte Behandlung chronischer und nicht chronischer Schmerzzustände, wobei hierunterdie erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception In letzter Zeit erschienen sind.

Klassische Opioide, wie beispielsweise das Morphin, sind bei der Therapie starker bis sehr starker Schmerzen wirksam. Als unerwünschte Begleitersdteinungen weisen sie jedoch unter anderem Atemdepression, Erbrechen, Sedieang. Obstipation sowie Toleranzentwicklung auf. Sie sind außerdem bei neuropathischen oder inzidentiellen Schmerzen, wie sie insbesondere bei Tumorpatienten häufig auftreten, weniger wirksam.

Aufgabe der vorliegenden Erfindung war es daher, neue Verbindungen zur Verfügung zu stellen, die sich als pharmazeutische Wirkstoffe in Arzneimitteln. vorzugsweise als pharmazeutische Wirkstoffe zur Schrnerzbekämpfung, vorzugsweise von chronischen oder neuropathischen Schmerzen, eignen und zur Behandlung oder Prophylaxe von neurodegenerativen Erkrankungen, vorzugsweise Morbus Alzheimer, Morbus Huntington oder Morbus Parkinson, Schlaganfall, cerebralem Infarkt, cerebraler lschämie, Himödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie. Tinnitus, Migräne, inflammatorlschen und/oder allergischen Reaktionen, Depressionen, seelischen Erkrankungen, Haminkontinenz, Juckreiz oder Diarrhoe oder zur Anxiolyse oder Anästhesie eingesetzt werden können.

Erfindungsgemäß wird diese Aufgabe durch Bereitstellung der substituierten Indol Verbindungen der nachstehenden allgemeinen Formel I, ggf. In Form ihrer Diastereomeren, reinen Enantiomeren, Racematen, nichtracemischen Gemischen der Enantiomeren oder Diastereomeren sowie jeweils ggf. in Form entsprechender Basen, Salze und Solvate geldst, wobei diese Verbindungen insbesondereeine ausgeprägte analgetische Wirkung aufweisen.

Gegenstand der Erfindung sind daher substituierte Indole der allgemeinen Formel 1,

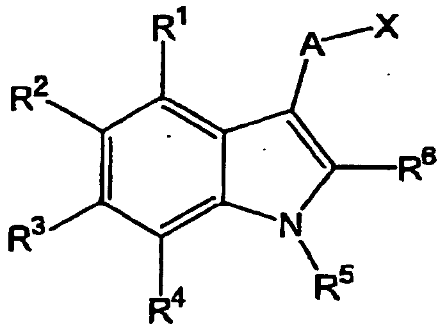

I

worin

$R^1$, $R^2$, $R^3$ und $R^4$, gleich oder verschieden, für einen linearen oder verzweigten, gesättigten oder ungesättigten aliphatischen $C_{1-10}$-Rest oder einen gesättigten oder ungesättigten cycloaliphatischen $C_{3-7}$-Rest, wobei jeder der vorstehend genannten Reste gegebenenfalls über eine Etherbrücke gebunden sein kann, oder Wasserstoff, ein Halogen oder eine Hydroxygruppe stehen,

$R^5$ für Wasserstoff. einen linearen oder verzweigten, gesättigten oder ungesättigten aliphatischen $C_{1-10}$-Rest oder einen gesättigten oder ungesättigten cycloaliphatischen $C_{3-7}$-Rest, einen Aryl- oder Hetemarylrest, wobei der Aryl-oder Heteroarylrest gegebenenfalls über eine $C_{1-6}$-Alkylengruppe gebunden sein kann, einen substituierten Sulfonylrest oder eine Gruppe der Formel $COR^7$ steht, wobei $R^7$ die unten angegebene Bedeutung hat,

$R^6$ für eine Gruppe der Formel $-COR^7$ steht, wobei der Rest $R^7$ die nachstehende Bedeutung hat,

$R^7$ für die Gruppe $OR^8$ steht, wobei der Rest $R^8$ die nachstehende Bedeutung hat,

$R^8$ für Wasserstoff oder für einen linearen oder verzweigten, gesättigten oder ungesättigten aliphatischen $C_{1-10}$.Rest steht,

A für eine Brücke mit einer der folgenden Formeln steht: $-(CH_2)_2$-oder $-(CH_2)_nNR^1$-, steht, worin n für 0, 1,2, oder 3 steht, $R^1$ die nachstehend angegebene Bedeutung hat und die Bindung zum Rest X immer zuletzt genannt ist

und X für den folgenden Rest steht worin der freie Strich die Bindung zur Brücke A symbolisiert

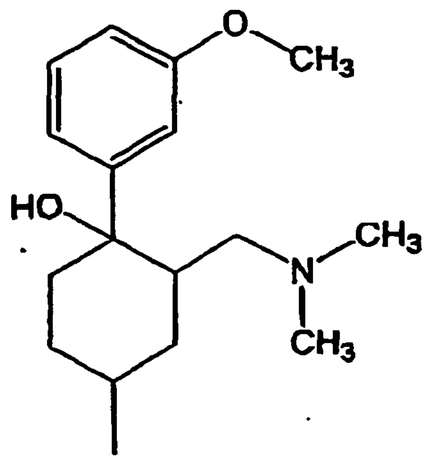

$R^{1'}$ für Wasserstoff oder für einen linearen oder verzweigten, gesättigten oder ungesättigten aliphatischen $C_{1-10}$-Rest steht,

gegebenenfalls in Form ihrer Racemate, ihrer reinen Stereoisomeren, insbesondere Enantiomeren oder Diastereomeren, oder In Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form Ihrer Salze, insbesondere der physiologisch verträglichen Salze, oder in Form ihrer Solvate, insbesondere der Hydrate.

Bevorzugt sind substituierte Indole der allgemeinen Famel 1, worin $R^2$ für einen linearen oder verzweigten, gesättigten oder ungesättigten aliphatischen $C_{1-3}$-Rest oder ein Halogen und $R^1$, $R^3$ und $R^4$ jeweils für Wasserstoff stehen, gegebenenfalls in Form ihrer Racemate, ihrer reinen Stereoisomeren, insbesondereEnantiomeren oder Diastereomeren, oder in Form von Mischungen der Stereoisomeren, insbesondere der Enantiomeren oder Diastereomeren, in einem beliebigen Mischungsverhältnis oder jeweils In Form Ihrer Sauren oder ihrer Basen oder in Form ihrer Salze, insbesondere der physiologisch verträglichen Salze, oder in Form ihrer Solvate, insbesondere der Hydrate.

Bevorzugt sind substituierte Indole der allgemeinen Formel I, worin $R^8$ für einen linearen oder verzweigten, gesättigten oder ungesättigten aliphatischen $C_{1-3}$-Rest oder ein Halogen und $R^1$, $R^2$ und $R^4$ jeweils für Wasserstoff stehen, gegebenenfalls In Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind substitulerte Indole der allgemeinen Formel I, worin $R^2$ und $R^3$, gleich oder verschieden, für einen linearen oder verzweigten, gesättigten oder ungesättigten aliphatischen $C_{1-3}$-Rest oder ein Halogen und $R^1$ und $R^4$ jeweils für Wasserstoff stehen, gegebenenfalls in Form ihrer Racemate, Ihrer reinen Stereoisomeren, insbesondere Enantiomeren oder Diastereomeren, oder in Form von Mischungen der Stereoisomeren, insbesondere der Enantiomeren oder Diastereomeren, In einem beliebigen Mischungsverhältris oder jeweils in Form ihrer Säuren oder ihrer Basen oder in Form ihrer Salze, insbesondere der physiologisch verträglichen Satze, oder in Form ihrer Solvate, insbesondere der Hydrate.

Bevorzugt sind substituierte Indole der allgemeinen Formel I, worin $R^1$ und $R^3$, gleich oder verschieden, für einen linearen oder verzweigten, gesättigten oder ungesättigten aliphatischen $C_{1-3}$-Rest oder ein Halogen und $R^2$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enanüomeren oder Diastereomeren, in einem bellebigen Miscl1ungsverhäJtnis oder jeweils in Form ihrer Säuren oder ihrer Basen oder in Form ihre- Salze, insbesondere der physiologisch verträglichen Salze, oder in Form ihrer Solvate, insbesondere der Hydrate.

Besonders bevorzugt sind substituierte Indole der allgemeinen Formel I, worin $R^2$ für einen Methylrest oder ein Chlor und $R^1$, $R^3$ und $R^4$ jeweils für Wasserstoff stehen, gegebenenfalls in Form ihrer Racemate, ihrer reinen Stereoisomeren, Insbesondere Enantlomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, insbesondere der physiologlsch verträglichen Salze, oder in Form ihrer Solvate, insbesondere der Hydrate.

Besonders bevorzugt sind substitulerte Indole der aligemeinen Formel 1, worin $R^3$ für einen Methylrest oder ein Chlor und $R^1$, $R^2$ und $R^4$ jeweils für Wasserstoff stehen, gegebenenfalls in Form ihrer Racemate, ihrer reinen Stereaisumeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, Insbesondere der physiologisch verträglichen Salze, oder in Form ihrer Solvate, insbesondere der Hydrate.

Besonders bevorzugt sind substituierte Indole der allgemeinen Formel 1, worin $R^2$ und $R^3$ jeweils für einen Methylrest oder ein Chlor und $R^1$ und $R^4$ jeweils für Wasserstoff stehen, gegebenenfalls in Form ihrer Facemate, ihrer reinen Stereoisomeren, insbesondere Enantiomeren oder Diastereomeren oder in Form von Mischungen der Stereoisomeren, insbesondere der Enantiomeren oder Diastereomeren, in einem beliebigen Mischungsverhältnis oder jeweils in Form Ihrer Säuren oder ihrer Basen oder In Form ihrer Satze, insbesondere der physiologisch verträglichen Salze, oder in Form ihrer Solvate, insbesondere der Hydrate.

Besonders bevorzugt sind substituierte lndole der allgemeinen Formel I, worin $R^1$ und $R^3$ jeweils für einen Methylrest oder ein Chlor und $R^2$ und $R^4$ jeweils für Wasserstoff stehen, gegebenenfalls in Form Ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, insbesondere der physiologisch verträglichen Salze, oder In Form Ihrer Solvate. Insbesondere der Hydrate.

Bevorzugt sind femer substituierte Indole der allgemeinen Formel I, worin $R^5$ für Wasserstoff steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder Ihrer Basen oder in Form ihrer Salze, insbesondere der physiologisch verträglichen Salze, oder In Form ihrer Solvate, insbesondere der Hydrate.

Bevorzugt sind femer substituierte Indole der allgemeinen Formel I, worin $R^6$ für eine Gruppe der Formel $COR^7$ steht, wobei $R^7$ für die Gruppe $OR^8$ und der Rest $R^8$ für Wasserstoff oder einen linearen oder verzweigten, gesättigten oder ungesättigten aliphatischen $C_{1-3}$-Rest, vorzugsweise eine Methyl- oder Ethylgruppe, steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, In einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Bevorzugt sind ferner substituierte Indole der allgemeinen Formel I, worin A für eine Brücke der folgenden Formel steht-$CH_2NR^{1'}$-, worin $R^{1'}$ für Wasserstoff oder eine lineare oder verzweigte, gesättigte oder ungesättigte aliphatische $C_{1-3}$-Gruppe steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form Ihrer Säuren oder ihrer Basen oder in Form ihrer Salze, insbesondere der physiologisch verträgllchen Salze, oder in Form ihrer Solvate, insbesondere der Hydrate.

Ganz besonders bevorzugt sind die folgenden substituierten Indole:

5-Methyl-3-([3'-(N.N-dimethylaminomethyl)-4'-hydroxy-4'-(3''-methoxyphenyl)-cyclohexylamino]-methyl)-1*H*-indol-2-carbonsäuremethylester,
4,6-Dimethyl-3-([3'-(N ,N-dimethylaminomethyl)-4'-hydroxy-4'-(3''-methoxyphenyl)-cyclohexylamino]-methyl}-1*H*-indol-2-carbonsäureethylester,
5-Chlor-3-{[3'-(N,N-dimethylaminomethyl)-4'-hydroxy-4'-(3''-methoxyphenyl)-cyclohexylamino]-methyl]-1*H*-indol-2-carbonsäureethylester,
4,6-Dichlor-3-{[3'-(N,N-dimethylaminomethyl)-4'-hydroxy-4'-(3''-methoxyphenyl)-cyclohexylamino]-methyl)-1*H*-indol-2-carbonsäureethylester,
4,6-Dimethyl-3-[[3'-(N,N-dimethylaminomethyl)-4'-hydroxy-4'-(3''-methoxyphenyl)-cyclohexyl-(N-methylamino)] -methyl]-1*H*-indol-2-carbonsäureethylester,
4,6-Dichlor-3-{[3'-(N,N-dimethylaminomethyl}4'-hydroxy-4'-(3''-methoxyphenyl)-cyclohexyl-(N-methylamino)]-methyl}-1*H*-indol-2-carbonsäureethylester,
5-Chlor-3-{[3'-(N,N-dimethylaminomethyl)-4'-hydroxy-4'-(3''-methoxyphenyl)-cyclohexyl-(N-methylamino)]-methyl}-1*H*-indol-2''-carbonsäureethylester,
5-Methyl-3-[[3'-(N,N-dimethylaminomethyl)-4'-hydroxy-4'-(3''-methoxyphenyl)-cyclohexylamino]-methyl}-1*H*-indol-2-carbonsäure,
5-Chlor-3-{[3'-(N,N-dimethylaminomethyl)-4'-hydroxy-4'-(3''-methoxyphenyl)-cyclohexylamino]-methyl}-1*H*-indol-2-carbonsäure,
4,6-Dimethyl-3-{[3'-(N,N-dimethylaminomethyl)4'-hydroxy-4'-(3''-methoxyphenyl)-cyclohexylamino]-methyl}-1*H*-indol-2-carbonsäure,
4,6-Dichlor-3-{[3'-(N,N-dimethylaminomethyl)-4'-hydroxy-4'-(3"-methoxyphenyl) cyclohexylamino]-mehyl}-1*H*-indol-2-carbunsäure,
1-tert-Butoxycarbonyl-4,6-dichlor-3-{[3'-(N,N-dimethylaminomethyl)-4'-hydroxy-4'-(3"-methoxyphenyl)-cyclohexylamino]-methyl}-indol-2-carbonsäureethylester,
4,6-Dichlor-3-{[3'-(N,N-dimethylaminomethyl)-4'-hydroxy-4'-(3"-methoxyphenyl)-cyclohexylamino]-methyl}-1-methyl-indol-2-carbonsäureethylester,
4,6-Dichlor-3-{[3'-(N,N-dimethylaminomethyl)-4'-hydroxy-4'-(3"-methoxyphenyl)-Cyclohexylamino]-methyl}-1-benzyl-indol-2-carbonsäureethylester.
5-Chlor-3-{[3'-(N,N-dimethylaminomethyl)-4'-(3"-methoxyphenyl)-cyclohexyamino]-methyl}-1-benzyl-indol-2-carbonsäureethylester,

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis oder jeweils in Form ihrer Säuren oder ihrer Basen oder in Form ihrer Salze, insbesondere der physiologisch verträglichen Salze, oder in Form ihrer Solvate, insbesondere der Hydrate.

Ein weiterer Gegenstand der vorlegenden Erfindung ist ein Verfahren zur Herstellung von substituierten Indolen der vorstehend angegebenen allgemeinen Formel I oder entsprechender Sterealsomeren, dadurch gekennzeichnet, daß man

A) gegebenenfalls ein Indol der Formel Y-$R^x$, worin $R^x$ für Wasserstoff oder eine Gruppe der Formel -$(CH_2)_nOH$ oder-$(CH_2)_nNR^{1'}H$ steht, worin n für 0, 1, 2 oder3 steht und $R^{1'}$ die vorstehend angegebene Bedeutung hat, und Y für einen Rest der allgemeinen Formel Y steht, worin der freie Strich die Bindung zum Rest $R^x$ symbolisiert

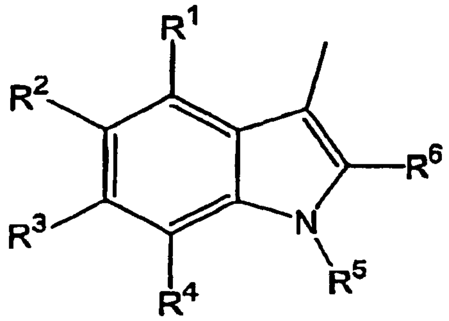

Y

und worin $R^1$, $R^2$, $R^3$, $R^4$, $R^5$ und $R^8$ die vorstehend genannte Bedeutung haben, derivatisiert, dadurch daß man

a) ein Indol der Formel Y-H mit einem N,N-disubstituierten Formamid, vorzugsweise N-Methyl-N-phenylformamid. In Gegenwart von Phosphoroxidchlorid, in einem geeigneten Lösungsmittel, vorzugsweise 1.2-Dichlorethan, zu dem entsprechenden Aldehyd der Formel Y-CHO umsetzt,
b) einen Aldehyd der Formel Y-CHO gemäß Schritt a) mit Hilfe von Raduktionsmitteln, vorzugsweise Natriumcyanoborhydrid oder $NaBH_2S_3$, in einem geeigneten lösungsmittel, gegebenenfalls in Gegenwart eines Puffers und unter Kühlung zu dem entsprechenden Alkohol der Formel Y-$CH_2$-OH umsetzt,
c) einen Alkohol der Formel Y-$(CH_2)_n$-OH gemäß Schritt b) oder A) mit einem Bromierungsmittel, vorzugsvmise $PBr_3$ oder $Ph_3PBr_2$, zu dem entsprechenden Bromid der Formel Y-$(CH_2)_n$-Br umsetzt.

und anschließend aufarbeitet und gegebenenfalls das Produkt reinigt,
C) gegebenenfalls eine Verbindung der Formel XR', worin X die vorstehend genannte Bedeutung hat und R' für eine funktionelle Gruppe steht, derivatisiert dadurch daß man

a) ein Keton der Formel X=O 1) mit Methoxymethyltriphenylphosphiniumchlorid unter Schutzgas in einem geeigneten Lösungsmittel vorzugsweise in Dimethylformamid, in Gegenwart von Natriumhydrid und anschließend mit Salzsäure oder 2) mit $Me_3S^+BF_4^-$ zu dem entsprechenden, um ein Kohlenstoffatom verlängerten Aldehyd X-CHO umsetzt,
b) einen Aldehyd der Formel X-CHO gemäß a) mit einem Reduktionsmittel, vorzugsweise Natriumborhydrid, in einem geeigneten Lösungsmittel, vorzugsweise einem Ethanol/Wasser-Gemisch zu dem entsprechenden Alkohol X-$CH_2$-OH umsetzt.

c) einen Alkohol $X-CH_2-OH$ gemäß b) oder der Formel X-OH mit einem Bromierungsmittel, vorzugsweise Triphenylphosphindibromid, in einem geeigneten Lösungsmittel, vorzugsweise Acetonitril, zu dem entsprechenden Bromid der Formel $X-CH_2-Br$ bzw. X-Br umsetzt,

d) ein Bromid der Formel $X-CH_2-Br$ gemäß c) mit einem Phosphin der Formel $PR''_3$, worin R" für einen organischen Rest steht, vorzugsweise für einen Phenylrest, in einem geeigneten Lösungsmittel, vorzugswelse Toluol, Ether, Tetrahydrofuran oder Aceton, unter Kühlung und Schutzgas zu dem entsprechenden Phosphoniumsalz $R''_3P^+-CHX^-$ umsetzt,

e) ein Bromid der Formel $X-CH_2-Br$ gemäß c) mit einem Phosphit der Formel $HP(O)(OR''')_2$, worin R"' für einen organischen Rest steht bei erhöhter Temperatur, vorzugsweise 200°C, zu dem entsprechenden Phosphonat $(R'''O)_2P(O)-CH_2-X$ umsetzt

und anschließend aufarbeitet und gegebenenfalls das Produkt reinlgt.

D) eine Verbindung der Formel $Y-R^x$ oder dessen Derivat aus dem Schritt A), worin Y die vorstehend genannte Bedeutung hat, mit einer Verbindung der Formel X-R' oder dessen Derivat aus Schritt C), worin X und R' die vorstehend genannte Bedeutung haben umsetzt, dadurch daß man

f) ein Amin der Formel $Y-(CH_2)_n-NHR^{1'}$ mit einem Bromid der Formel X-Br in Gegenwart eines geeigneten Katalysators, vorzügsweise Caesiumcarbonat, in einem geeignten Lösungsmittel, vorzugsweise Dimethylformamid, unter Ausbildung einer Aminobrücke umsetzt,

g) ein Bromid der Formel $Y-(CH_2)_n-Br$ mit einem Amin der Formel $X-NHR^{1'}$ in Gegenwart eines geeigneten Katalysators, vorzugsweise Caesiumcarbonat. In einem geeignten Lösungsmittel, vorzugsweise Dimethylformamid, unter Ausbildung einer Aminobrücke umsetzt,

h) einen Aldehyd der Formel Y-CHO mit einem Amin der Formel $X-NHR^{1'}$ in Gegenwart eines geeigneten Reduktionsmittels, vorzugsweise Natriumcyanoborhydrid und Natriumtriacetoxyborhydrid, In einem geeigneten Lösungsmittel, vorzugsweise einem Gemisch aus Tetrahydrofuran und 1,2-Dichlorethan, unter Ausbildung einer $-CH_2-NR^{1'}$-Brücke umsetzt,

i) einen Aldehyd der Formel Y-CHO mit einem Phosphoniumsatz $R''_3P^{+-}CHX^-$, worin R" die vorstehend genannte Bedeutung hat, unter Schutzgas in Gegenwart geeigneter Katalysatoren in einem geeigneten Lösungsmittel vorzugsweise in Gegenwart von Natriummethanolat in einem Gemisch aus Hexan, Diethylether und/oder Diisopropylether oder in Gegenwart von Natriumhydrid. Kalium-tert-butylat oder einem Lithiumamid in Dimethylformamid oder Dimethylsulfoxid, unter Ausbildung einer CH=CH-Brücke umsetzt oder

j) einen Aldehyd der Formel Y-CHO mit einem Phosphonat der Formel $(R'''O)_2P(O)-CH_2-X$, worin R"' die vorstehend genannte Bedeutung hat, unter Schutzgas in Gegenwart geeigneter Katalysatoren, vorzugsweise Natriummethanolat Natriumhydroxid, Kaliumhydroxid, Natrlumhydrld, Kallum-tert-butylat oder einem Lithiumamid, in einem geeigneten Lösungsmittel, vorzugsweise Dimethylformamid, Dimethylsufoxid, Diethylether. Tetrahydrofuran, unter Ausbildung einer -CH=CH-Brücke umsetzt.

k) gegebenenfalls die -CH=CH-Brücke aus dem Schritt i) oder j) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C unter Ausbildung einer $-CH_2-CH_2$-Brücke hydriert,

und anschließend aufarbeitet und gegebenenfalls das Produkt reinigt,

E) gegebenenfalls einen Indol-2-carbonsäureester der Formel Y-A-X. worin Y, A und X die vorstehend genannte Bedeutung haben, wobei $R^6$ in Y für eine Gruppe der Formel $COR^7$ steht, worin $R^7$ für die Gruppe $OR^8$ steht und $R^8$ die vorstehend genannte Bedeutung mit Ausnahme von Wasserstoff hat, in Gegenwart einer Base, vorzugsweise Kalium oder Natrium-Hydroxid, in einem geeigneten Lösungsmittel, vorzugsweise einem Aikohot/Wassergemisch, besonders bevorzugt in einem Methanol/ oder Ethanol/Wassergemisch, verseift und anschießend aufarbeitet und gegebenenfalls die Indol-2-carbonsäure der Formel Y-A-X worin $R^8$ in Y für eine Gruppe der Formel $COR^7$ steht, worin $R^7$ für die Gruppe $OR^8$ und $R^8$ für Wasserstoff steht, reinigt.

Die zum Einsatz kommenden Lösungsmittel und Umsetzungsbedingungen entsprechen den für diese Typen von Reaktionen üblichen Lösungsmitteln und Umsetzungsbedingungen.

Die Indole der Formel $Y-R^x$ können nach der Fischer-Indol-Synthese, die dem Fachmann aus Gray et al., J. Med. Chem. 34, 1283 (1991); lishii, Chem. Pharm. Bull. 21,1481 (1973) und Salituro et al., J. Med. Chem. 35, 1791 (1992) und der darin zitierten Literatur bekannt ist, hergestellt werden.

Gegebenenfalls sind Derivatisierungsreaktionen erforderlich, die die funktionellen Gruppen zur Verknüpfung des Indol-Gerüstes über die Brücke A mit dem Rest X einführen. Die Verselfung von Estern erfolgt nach den üblichen,

dem Fachmann bekannten Methoden. Die übrigen Umsetzungen sind aus der folgenden Literatur und der darin zitierten Literatur bekannt die Formyllerung aus R. di Fablo et al., J. Med. Chem. 40, 841 (1997), der Internationalen Patentanmeldung WO 9510517 und der US-amenkanischen Patentanmeldung US 5922752, die Reduktion von Aldehyden zu Alkoholen aus Synthesis 526 (1972) und Synthesis 135 (1975) und die Umsetzung von Alkoholen zu Bromiden aus J. Am Chem. Soc. 48,1080 (1928); J. Chem. Soc., 636 (1943); Org. Synth. Coll., Vol. 2,358 (1943); Liebigs Ann. Chem. 626, 26 (1959); J. Am. Chem. Soc. 86, 964 (1964); J. Am. Chem. Sec. 99,1612 (1977).

Verbindungen mit Resten, die unter den allgemeinen Rest $X^2$ fallen, sind aus der deutschen Patentanmeldung P 3217639 bekannt.

Aus der Literatur sind Verbindungen X-OH, X-$NHR^{1'}$, X=O und X-$CO(CH_2)_pOH$ bekannt oder sie können aus bekannten, am Markt erhältlichen Verbindungen nach üblichen, dem Fachmann bekannten Methoden oder nach Methoden, wie sie in der deutschen Patentanmeldung P100494811 beschrieben sind, hergestellt werden.

Gegebenenfalls sind Derivatisierungsreaktionen erforderlich, die die funktionellen Gruppen zur Verknüpfung des Restes X mit dem IndolGerüst über die Brücke A einführen. Diese Umsetzungen könnennach üblichen, dem Fachmann bekannten Methoden erfolgen und sind aus der folgenden literatur und der darin zitierten Literatur bekannt die Umsetzung von Ketonen zu um ein Kohlenstoff verlängerten Aldehyden aus der deutschen Patentanmeldung P 100494811; J. Nat Prod. 44, 557 (1981) und Synth. Commun. 12,613 (1982), die Reduktion von Aldehyden zu Alkoholen aus der deutschen Patentanmeldung P 100494811 und Chem. Commun. 535 (1975), die Umsetzung von Alkoholen zu Bromiden aus J. Am Chem. Soc. 48,1080 (1926);J. Chem. Soc., 636 (1943); Org. Synth. Coll., Vol. 2, 358 (1943): Liebigs Ann. Chem. 626, 26 (1959); J. Am. Chem. Soc. 86, 964 (1964); J. Am. Chem. Soc. 99, 1612 (1977), die Darstellung der Phosphonate und der Phosphoniumsalze ist aus M. Schlosser, Top. Stereochem. 5,1 (1970); R. Broos, D. Tavernier, M. Anteunis, J. Chem. Educ. 55, 813 (1978); G. Wittig. Angew. Chem. 92, 671 (1980); H.J. Bestmann; Pure Appl. Chem. 52, 771 (1980) und L. Homer, H. Hoffmann, H.G. Wippel, G. Klahre: Chem. Ber. 92, 2499 (1959); J. Gillois. G. Gudlerm, M. Savignac, E. Stephan, L Vo Quang, J. Chem. Educ. 57. 161 (1980); B.A. Arbusov; Pure Appl. Chem. 9,307 (1964); A.K. Bhattacharya, G. Thyagarajan; Chem. Rev. 81,415 (1981).

Die Verknüpfung des Restes X mit dem Indol-Gerüst über die Brücke A kann nach üblichen, dem Fachmann bekannten Methoden erfolgen und ist aus der folgenden Literatur und der jeweils darin zitierten Literatur bekannt die Umsetzung von Carbonsäuren mit Alkoholen oder Aminen In Gegenwart von Dicyclohexylcarbodiimid aus W. König, R. Geiger, Chem. Ber. 103, 788 (1970), die Umsetzung von Carbonsäuren mit Alkoholen in Gegenwart von 1(Mesitylen-2-sulfonyl)-3-nitro-1,2,4-triazol aus Tetrahedron 38, 3075 (1980), die Veretherung aus Tetrahedron 35, 2169 (1979), Tetrahedron Lett. (1973), 21; Synthesis. 434 (1974); J. Org. Chem. 52,4665 (1987), die reduktive Aminierung aus Org. React. 3, 174 (1948); J. Am. Chem. Soc. 91, 3996 (1969): Org. Prep. Proced. Int 11, 201 (1979); Org. Prep. Proced. Int. 17,317 (1985), die Wittig oder Wittig-Homer-Emmons-Reaktion aus G. Wittig. Angew. Chem. 92, 671 (1980); H.J. Bestmann; Pure Appl. Chem. 52, 771 (1980) und L.Homer, H. Hoffmann, H.G. Wippel, G. Klahre; Chem. Ber. 92, 2499 (1959); J. Gillois, G. Guillerm, M. Savignac, E. Stephan, L. Vo Quang; J. Chem. Educ. 57, 161 (1980); B.A. Arbusov; Pure Appl. Chem. 9, 307 (1964); A.K Bhattacharya. G. Thyagarajan; Chem. Rev. 81, 415 (1981) und die Hydrierung aus Synthesis (1978). 329; J. Org. Chem. 34, 3684 (1969); J. Am. Chem. Soc. 91, 2579 (1969).

Die entsprechenden Literaturbeschreibungen werden hiermit als Referenz eingeführt und gelten als Teil der Offenbarung.

Die erfindungsgemäßen substituierten IndolVerbindungen der allgemeinen Formel I sowie entsprechende Stereoisomere könnensowohl in Form ihrer freien Basen als auch in Form entsprechender Salze Isoliert werden.

Die freien Basen der jeweiligen erfindungsgemäßen Verbindungen der allgemeinen Formel I sowie entsprechender Stereoisomere können durch Umsetzung mit einer anorganischen oder organischen Saure, vorzugsweise mit Salzsäure, Bromwasserstoffsäure, Schwefelsäure. Phosphorsäure, Methansulfonsäure, p-Toluolsulfonsäure, Kohlensäure, Ameisensäure, Essigsäure, Oxalsäure, Bemsteinsäure, Weinsäure, Mandelsäure, Fumarsäure, Michsäure, Citronensäure, Glutaminsäure oder Asparaginsäure, in die entsprechenden physiologisch verträglichen Salze überführt werden.

Die freien Basen der jeweiligen erfindungsgemäßen Verbindungen der allgemeinen Formel I sowie entsprechender Stereoisomere können bevorzugt durch Versetzen der in einem geeigneten organischen Lösungsmittel, wie z.B. Butan-2-on (Methylethylketon), gelösten erfindungsgemäßen Verbindungen der allgemeinen Formel I oder entsprechender Stereoisomere als freie Basen mit Trimethylsilylchlorid (TMSCI) in die entsprechenden Hydrochloride Überführt werden.

Die freien Basen der jeweiligen erfindungsgemäßen Verbindungen der allgemelnen Formel I und entsprechender Stereoisomere können mit der freien Säure oder einem Salz eines Zuchersatzstoffes, wie z.B. Saccharin, Cyclamat oder Acesulfam, in die entsprechenden physiologisch verbüglichen Salze überführt werden.

Die erfindungsgemäßen substituierten IndolVerbindungen der allgemeinen Formel I und entsprechende Stereoisomere können ggf.,ebenso wie die entsprechenden Säuren, die entsprechenden Basen oder Salze dieser Verbindungen, auch in Form ihrer Solvate, vorzugsweise ihrer Hydrate, erhalten werden.

Sofern die erfindungsgemäßen substituierten Indo-Verbindungen der allgemeinen Formel I nach dem erfindungsgemäßen Herstellungsverfahren in Form von Stereoisomeren, vorzugswelse In Form ihrer Racemate oder anderer Mischungen ihrer verschiedenen Enantiomeren und/oder Diastereomeren erhalten werden, können diese nach Oblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLG und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+) -Weinsäure, (-)-Weinsäure oder (+)-10-Camphersulfonsäure, gebildete diastereomere Salze voneinander getrennt werden.

Die erfindungsgemäßen substituierten IndolVerbindungen der allgemeinen Formel 1 und entsprechende Stereoisomere sowie Jeweils die entsprechenden Säuren, Basen, Salze und Solvate sind toxikologisch unbedenklich und eignen sich daher als phannazeutische Wirkstoffe in Arzneimitteln.

Ein weiterer Gegenstand dervorfiegenden Erfindung sind daher Arzneimittel, die wenigstens eine erfindungsgemäße substituierte IndolVerbindung der allgemeinen Fonnel 1. gegebenenfalls in Form ihres Racemates, ihres reinen Stereoisomeren, insbesondere Enantiomeren oder Diastereomeren, oder in Form von Mischungen der Stereoisomeren, Insbesondere der Enantiomeren oder Diastereomeren, in einem beliebigen Mischungsverhältnis oder jeweilsin Form ihrer Säure oder ihrer Base oder in Form ihres Salzes, insbesondere eines physiologisch verträglichen Salzes, oder in Form ihres Solvates, insbesondere des Hydrates sowie gegebenenfalls physiologisch verträgliche Hilfsstoffe enthalten. Selbstversändlich können die erfindungsgemäßen Arzneimittel auch Mischungen aus zwei oder mehr der vorstehend genannten Verbindungen aufweisen.

Sofem die erfindungsgemäßen substituierten Indol Verbindungen der allgemeinen Formel 1 oder deren entsprechende physiologisch verträgliche Basen, Salze oder Solvate chiral sind, können sie -wie bereits aufgeführt- bevorzugt in Form ihrer Racemate, ihrer reinen Enantiomeren, ihrer reinen Diastereomeren oder in Form eines Gemisches aus wenigstens zwei der vorstehend genannten Stereoisomeren in dem erfindungsgemäßen Arzneimittel vorliegen.

vorzugsweise eignen sich die erfindungsgemäßen Arzneimittel zur Bekämpfung von Schmerzen, vorzugsweise von chronischen oder neuropathischen Schmerzen. und zur Behandlung oder Prophylaxe von neurodegenerativen Erkrankungen, vorzugsweise Morbus Alzheimer, Morbus Huntington oder Morbus Parkinson, Schlaganfall, cerebralem Infarkt, cerebraler Ischämie, Himödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tmnitus, Migräne, inflammatorischen und/oder allergischen Reaktionen, Depressionen, seelischen Erkankungen, Haminkontinenz, Juckreiz oder Diarrhoe oder zur Anxiolyse oder Anästhesie.

Die Verwendung wenigstens einer substituierten IndolVerbindung der allgemeinen Formel I, gegebenenfalls in Form ihres Racemates, ihres reinen Stereoisomeren, insbesondere Enantiomeren oder Diastereomeren, oder in Form von Mischungen der Stereoisomeren, Insbesondere der Enantiomeren oder Diastereomeren, in einem beliebigen Mischungsverhältnis oder jeweils in Form ihrer Säure oder ihrer Base oder in Form ihres Salzes, insbesondere eines physiologisch verträglichen Salzes, oder in Form ihres Solvates, insbesondere des Hydrates zur Herstellung eines Arzneimittels zur Bekämpfung von Schmerzen, vorzugsweise von chronischen oder neuropathischen Schmerzen, zur Behandlung oder Prophylaxe von neurodegenerativen Erkrankungen, vorzugsweise Morbus Alzheimer, Morbus Huntington oder Morbus Parkinson. Schlaganfall, cerebralem Infarkt, cerebraler Ischämie, Himödem, Unterversorgungszuständen des zentralen Nervensystems, vor allem Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tinnitus. Migräne, inflammatorischen und/oder allergischen Reaktionen, Depressionen, seelischen Erkrankungen, Haminkontinenz, Juckreiz oder Diarrhoe oder zur Anxiolyse oder Anasthesia ist ebenfalls Gegenstand der vorliegenden Erfindung.

Die erfindungsgemäßen Arzneimittel können als flüssige, halbfeste oder feste Arzneiformen, beispielsweise in Form von injektionslösungen. Tropfen Säften, Sirupen, Sprays. Suspensionen, Tabletten, Patches, Kapseln, Pflastern. Zäpfchen, Salben, Cremes. Lotionen. Gelen, Emulsionen, Aerosolen oder in multipartikulärer Form, beispielsweise in Form von Pellets oder Grarulaten, vorliegen und als solche auch verabreicht werden.

Neben wenigstens einer erfindungsgemäßen substituierten Indol-Verbindung der allgemeinen Formel I, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnls, oder jeweils in Form ihrer Säure oder Ihrer Base oder in Form ihres Satzes, insbesondere eines physiologisch verträglichen Salzes, oder In Form Ihres Solvates, insbesondere des Hydrates, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln. Verdünnungsmitteln, oberflächenaktiven Stoffen, Fabstoffen, Konservierungsstoffen, Sprengmittein, Gleitmitteln, Schmiermittein, Aromen und Bindemitteln.

Die Auswahl der physiologisch verträglichen Hilfsstoffe sowie die einzusetzenden Mengen derselben hängt

davon ab, ob das Arzneimittel oral, subkutan, parenteral, intravenös, intraperitoneal, intradermal, intramuskulär, Intranasal, buccal, rectal oder örtlich, zum Beispiel auf Infektionen ander Haut, der Schleimhäute und an den Augen, appliziert werden soll. Für die orale Applikation eignen sich bevorzugt Zubereitungen in Form von Tabletten. Dragees, Kapseln, Grarulaterl. Pellets, Tropfen, Säften und Sirupen, für die parenterale, topische und inhalative Applikation Lösungen, Suspensbnen, leicht rekonstituierbare Trockenzubereltungen sowie Sprays.

Erfindungsgemäße Verbindungen der allgemeinen Formel I, gegebenenfalls in Form Ihres Racemates, ihres reinen Stereoisomeren, insbesondere Enantiomeen oder Diastereomeren, oder in Form von Mischungen der Stereoisomeren. insbesondere der Enantiomeren oder Diastereomeren, in einem beliebigen Mischungsverhältnis, oder jeweils in Form ihrer Säure oder Ihrer Base oder in Form ihres Salzes, insbesondere eines physiologisch verträglichen Salzes, oder in Form ihres Solvates, Insbesondere des Hydrates, In einem Depot in gelöster Form oder in einem Pflaster, gegebenenfalls unter Zusatz von die Hautpenetration fördemden Mitteln, sind geeignete perkutane Applikationszubereitungen. Oral oder perkutan anwendbare Zubereitungsformen können die erfindungsgemäßen Verbindungen der allgemeinen Formel 1, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem.beliebigen Mischungsverhältnis, oder jeweils in Form ihrer Säure oder ihrer Base oder in Form ihres Salzes, insbesondere eines physiologisch verttglichen Salzes, oder in Form Ihres Solvates, insbesondere des Hydrates, auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences. 17. Edition, Mack Publishing Company, Easton. Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen substituierten Indol-Verbindung der allgemeinen Formel 1, gegebenenfalls in Form ihres Racemates, ihres reinen Stereoisomeren, insbesondere Enantiomeren oder Diastereomeren, oder In Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihes Salzes, insbesondere eines physiologisch verträglichen Satzes, oder in Form ihres Solvates, insbesondere des Hydrates, kann variieren und ist beispielsweise abhängig vom Gewicht oder Aiter des Patenten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer erfindungsgemäßen substitulerten Indol-Verbindung der allgemeinen Formel 1, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, appliziert.

Die Untersuchung der erfindungsgemäßen Verbindungen auf analgetische Wirksamkeit wurde im Phenylchinon-induzierten Writhing-Test, modifiziert nach I.C. Hendershot, J. Forsaith in J. Pharmacol, Exp. Ther. 125, 237-240 (1959), an der Maus durchgeführt Die entsprechende Literaturbeschreibung wird hiermit als Referenz eingeführt und gilt als Teil der Offenbarung.

Dazu wurden männliche NMRI-Mäuse mit einem Gewicht von 25-30 g verwendet. Gruppen von jeweils 10 Tieren pro Verbindungsdosls erhielten 10 Minuten nach intravenöser Gabe der Prüfverbindungen 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden anschlleßend einzeln in Beobachtungskäfige gesetzt Mittels eines Drucktastenzählers wurde die Anzahl der Schmerz-induzierten Streckbewegungen (sogenannte Writhing-Reaktionen = Durchdrücken des Körpers mit Abstrecken der Hinterextremiläten) 5-20 Minuten nach der Phenylchinon-Gabe ausgezählt. Als Kontrolle wurden Tiere mitgeführt, die nur physiologische Kochsalzösung mit Phenylchinon erhielten.

Die erfindungsgemäßen Verbindungen wurden In der Standarddosis von 10 mg/kg getestet Die prozentuale Hemmung (% Hemmung) der Writhlngreaktionen durch eine Verbindung wurde nach folgender Formel berechnet:

$$\%\ \text{Hemmung} = 100 - \left[ \frac{\text{Writhingreaktion behan. Tiere}}{\text{Writhingreaktion Kontrolle}} \times 100 \right]$$

Im folgenden wird die Erfindung anhand von Beispielen erläutert. Diese Erläuterungen sind lediglich beispielhaft und schränken den allgemeinen Erfindungsgedanken nicht ein.

**Beispiele**

**Die Ausbeuten der erfindurgsgemäßen Belsplelverbindungen wurden nicht optimiert.**

**Beispiel 1:**

**Synthese von 5-Methyl-3-{[3'-(N,N-dimethylaminomethyl)-4'-hydroxy-4'-(3"-methoxyphenyl)-cyclohexylamino]-methyl}-1*H*-indol-2-carbonsäuremethylester**

5-Methyl-3-formyl-1H-indol-2-carbonsäuremethylester(462 mg, 2 mmol) und 4-Amino-2-(N,N-dlmethylaminumethyl)-1-(3'-mathoxyphenyly)-cyclohexan-1-ol (556 mg, 2 mmol) wurden In trockenem THF (5 ml) und 1,2-Dichlorethan (15 ml) gelöst, mit geglühtem Natriumsulfat (2 g) versetzt und 48 h bei Raumtemperatur geführt Anschließend wurde Natriumtriacetoxyborhydrid (600 mg, 2,8 mmol) hinzugegeben und weitere 4 h gerührt. Das Natriumsulfat wurde abgesaugt, das Lösungsmittel im Vakuum abdestilliert, der Rückstand in Essigester (10 ml) gelöst und mit 0,5 N HCl (10 ml) versetzt. Die Phasen wurden getrennt, die wäßrige Phase mit Esslgester extrahiert (4 x 10 ml) und anschließend mit Natriumhydrogencarbonat auf pH 8 gebracht und nochmals mit Essigester (3 x20 ml) extrahiert Nach dem Abdestillieren des lösungsmittels wurde das Amin (810 mg, 82 %) als fabloser Feststoff erhalten. Der Schmelzpunkt lag bei 81-84 °C.

**Beispiel 2:**

**Synthese von 4,6-Dimethyl-3-{[3'-(N,N-dimethylaminomethyl)-4'-hydroxy-4'-(3"-methoxyphenyl)-cyclohexylamino]-methyl}-1*H*-indol-2-carbonsäureethylester**

Die Darstellung erfolgte analog zu Beispiel 1.4,6Dimethyl-3-formyl-1H-indol-2-carbonsäureethylester (981 mg, 4 mmol) und 4-Amino2-(N,N-dimemylaminomethyl)-1-(3'-methoxyphenyl)-cyclohexan-1-ol (1,112 g, 4 mmol) wurden in THF (16 ml) und 1,2-Dichlorethan (60 ml) gelöst. Dazu wurde getrocknetes Natriumsulfat (4 g) gegeben und 24 h gerührt, anschließend wurde Natriumtriacetoxyborhydrid (2,4 g. 11.2 mmol) hinzugefügt und weitere 2 h gerührt. Zur Aufarbeitung wurde das Natriumsulfat abgesaugtund mit Ethylacetat (3 x 15 ml) gewaschen. Die Lösung wurde mit 1N HCl (15 ml) versetzt und 5 min gerührt. Anschließend an die Phasentrennung wurde die organische Phase mit 1 N HCl (2× 5 ml) und die wäßrige Phase mit Ethylacetat (2×10 ml) extrahiert. Die wäßrige Phase wurde mit Natriumhydrogencarbonat auf pH 8 gebracht und emeut mit Ethylacetat extrahiert (4 x 50 ml). Nach dem Trocknen der organischen Phase und Abdestillieren des Lösungsmittels wurden 1.99g (98 %) des Kuppungsproduktes erhalten. Der Schmelzpunkt der Verbindung lag bei 79-82 °C,

**Beispiel 3:**

**Synthese von 5-Chlor-3-{[3'-(N,N-dimethylaminomethyl)4'-hydroxy-4'-(3"-methoxyphenyl)-cyelohexylamino] methyl}-1*H*-indol-2-carbonsäureethylester**

Die Darstellung erfolgte analog zu Beisplel 1. Antelle von 5-Methyl-3-formyl-1H-indol-2-carbonsäuremethylester wurde 5-Chlor-3-formyl-1 H-Indol-2 carbansäureethylester zur Reaktion gebracht Der Schmelzpunkt der Verbindung lag bei 84-87°C.

**Beispiel 4:**

**Synthese von 4,6-Dichlor-3{[3'-(N,N-dimethylaminomothyl)-4-hydroxy-4'-(3"-methoxypheny)-cyclohexylamino]-methyl}-1*H*-indol-2-carbonäureathylester**

Die Darstellung erfolgte analog zu Belspiel 1. 4,6Dichlor-3-formyl-1H-indol-2 carbonsäureethylester und 4-Amino-2-(N,N-dimethylaminomethyl)-1-(3'-methoxyphenyl)-cyclohexan-1-ol wurden in THF mit einem Übemrhuß an getrocknetem Natriumsulfat 72 h bei 20°C gerührt Ohne Isollerung des Imins wurde anschließend die Reduktion mit Natriumtriacetoxyborhydrid innerhalb von 24 h bei 20°C durchgeführt Die Ausbeute betrug nach säulenchromatographischer Reinigung 90%. Der Schmelzbereich der Verbindung lag bei 71-81°C.

**Beispiel 5:**

**Synthese von 4,6-Dimethyl-3-{[3'-(N,N.dimethylaminomethyl)-4'-hydroxy-4'. (3"-methoxyphenyl)-cyclohexyl-(N-methylamino)]-methyl}-1*H*-indol-2-carbonsäureethylestor**

4,6-Dimethyl-3-formyl-1H-indol-2-carbonsäureethylester und 2-(N,N-Dimethylaminomethyl)-1-(3'-methoxyphenyl)-4-(N-methylamino)-cyclohexan-1-ol wurden analog zu Beispiel 6 umgesetzt. Der Schmelzpunkt der Verbindung lag bei 75-77°C.

**Beispiel 6:**

**Synthese von 4,6-Dichlor-3-{[3'N,N-dimethylaminomethyl)-'4-hydroxy-4'-(3"-methoxyphenyl)-cyclohexyl-(N-methylamino)]-methyl}-1*H*-Indol-2-carbonsäureethylester**

4,6-Dichlor 3-formyl-1H-indol-2-carbonsäureethylester (172 mg, 0,6 mmol) und 2-(N,N-Dimethylaminomethyl)-1-(3'-methoxyphenyl)-4-(M-methylamino)-cyclohexan-1-ol (175 mg, 0,6 mmol) wurden in trockenem Tetrahydrofuran (10 ml) und trockenem 1,2-Dichlorothan (10 ml) gelöst mit gaglühtem Natriumsulfat (2 g) und Natriumtriacetoxyborhydrid (178 mg, 0,84 mmol) vesetzt und 3 Tage gerührt. Zur Aufarbeitung wurde das Lösungsmittel im Vakwm abdestilliert; der Rückstand mit Ethylacetat (10 ml), Wasser (10 ml) und 10 %ige Schwefelsäure (2 ml) versetzt, die Phasen getrennt, die saure wäßrige Phase mit Ethylacetat (3×10 ml) extrahiert, mit Natriumhydrogencarbonat auf pH 7-8 gebracht und nochmals mit Ethylacetat (3 × 10 ml) extrahiert. Der Extrakt lieferte nach dem Trocknen und Abdestillieren des Lösungsmittels 172 mg (51 %) eines farblosen Feststoffes mit einem Schmelzpunkt bel 82-84 °C.

**Beispiel 7:**

**Synthese von 5-Chlor-3-{[3'-(N,N-dimethylaminomethyl)-4'-hydroxy-4'-(3"-methoxyphenyl)-cydohexyl-(N-methylamino)]-methyl}-1*H*-indol,2-carbonsäureethylester**

5-Chlor-3-formyl-1H-Indol-2-carbonsäuroethylester und 2-(N,N-Dimethylaminomethyl)-1-(3'-methoxyphenyl)-4-(N-methylamino)-cyclohexan-1-ol wurden analog zu Beispiel 6 umgesetzt. Der Schmelzpunkt der Verbindung lag bei 72.74°C.

**Beispiel 8 (REFERENZ) :**

**Synthese von 4,6-Dichlor-3-[3'-hydroxy-3'-(3"-methoxyphenyl)-piperidin-1'-ylmethyl]-1*H*-indol-2-carbonsäureethytester**

4,6-Dichlor-3-formyl-1H-indol-2-carbonsäureethylester (454 mg, 1,59 mmol) wurde in THF (15 ml) und 1,2-Dichlorethan (15 ml) gelöst. 3-(3'-Methoxyphenyl)-piperidin-3-ol (330 mg, 1,59 mmol) und Natriumtriacetoxybortydrid (477 mg. 2,22 mmol) wurden hinzugefügt und 20h bei Raumtemperatur gerührt. Das Lösungsmittel wurde abdestilliert, der Rückstand In Ethylacetat (10 ml) gelöst mit 10 %iger. Schwefelsäure (2,5 ml) und Wasser (10 ml) versetzt Die Phasen wurden getrennt und die wäßrige Phase mit Ethylacetat extrahiert (3x 10 ml). Die wäßrige Phase wurde mit Natriumhydrogencarbonatiösung (15 ml) auf pH 7-8 gebracht und nochmals mit Ethylacetat extrahiert (3×10ml). Nach dem Trocknen der organische Phase und Abdestillieren des Lösungsmittels wurden 530 mg (70 %) eines farblosen Feststoffes erhalten. Der Schmelzpunkt der Verbindung lag bei 59 52°C.

**Beispiel 9 (REFERENZ)**:

**Synthese von 4,6-Dimethyl-3-[3'-hydroxy-3'-(3"-methoxyphenyl)-piperidin-1'-ylmethyl]-1 *H*-Indol-2-carbonsäureethylester**

4,6-Dimethyl-3-formyl-1H-indol-2-carbonsäureethylester (355 mg, 1,45 mmol) wurde in THF (15 ml) und 1,2-Dichlorethan (15 ml) gelöst. 3-(3'-Methoxyphenyl)-piperidin-3-ol (300mg. 1,45 mmol) und Natriumtriacetoxybortydrid (429 mg, 2,03 mmol) wurden hinzugefügt und 48 h bei Raumtemperatur gerührt. Das Lösungsmittel wurde abdestilliert, der Rückstand in Ethylacetat (10 ml) gelöst mit 10 %-iger Schwefelsäure (2 ml) und Wasser (10 ml) versetzt, die Phasen getrennt und die wäßrige Phase mit Ethylacetat extrahiert (3x 10 ml). Die wäßrige Phase wurde mit Natriumhydrogencarbonatlösung (10 ml) auf pH 7-8 gebracht und nochmals mit Ethylacetat extrahiert (4x 10 ml). Nach Trocknen der organische Phase und Abdestillieren des Lösungsmittels wurden 370 mg (59 %) eines farblosen Feststoffes mit einem Schmelzpunld bei 70-72°C erhalten.

**Beispiel 10 (REFERENZ)** :

**Synthese von 5-Methyl-3-[3'-hydroxy3'-(3"-methoxyphenyl)-piperidin-1'-ylmethyl]-1*H*-indol-2 carbonsäure-ethylester**

5-Methyl-3-formyl-1H-indol-2-carbonsäureethylester (355 mg, 1,45 mmol) wurde in THF (15 ml) und 1,2-Dichlorethan (15 ml) gelöst. 3-(3'-Methoxyphenyl)-piperidin-3-ol (300 mg, 1,45 mmol) und Natriumtriacetoxyborhydrid (429 mg, 2,03 mmol) wurden hinzugefügt und 48 h bei Raumtemperatur gerührt. Das Lösungsmittel wurde abdestilliert, der Rückstand in Ethylacetat (10 ml) gelöst, mit 10 %iger Schwefelsäure (2 ml) und Wasser (10 ml) versetzt, die Phasen getrennt und die wäßrige Phase mit Ethylacetat extrahiert (4x10 ml). Die wäßrige Phase wurde mit Natriumhydrogencarbonatiösung (10 ml) auf pH 7-8 gebracht und nochmals mit Ethylacetat extrahiert (4 x 10 ml). Nach demTrocknen der organischen Phase und Abdestillieren des Lösungsmittels wurden 364 mg (60 %) eines farblosen Feststoffes mit einem Schmelzpunkt bei 50-52°C erhalten.

**Beispiel 11 (REFERENZ):**

**Synthese von 5-Chlor-3-[3'hydroxy-3'-(3''methoxyphenyl)-piperidin-1'-ylmethyl]-1 *H*-indol-2-carbonsäureethylester**

5-Chlor-3-formyl-1 H-Indol-2-carbonsäureethylester (439 mg, 1,74 mmol) wurde in THF (15 ml) und 1,2-Dichlorathan (15 ml) gelöst. 3-(3'-Methoxyphonyl)-piperidin-3-ol (361 mg, 1,74 mmol) und Natriumtriacetoxyborhydrid (516 mg, 2,43 mmol) wurden hinzugefügt und 48 h bel Raumtemperatur gerührt. Das Lösungsmittel wurde abdestilliert, der Rückstand in Ethylacetat (10 ml) gelöst mit 10 %iger Schwefelsäure (3 ml) und Wasser (10 ml) versetzt, die Phasen getrennt und wäßrige Phase mit Ethylacetat extrahiert (4 x 10ml). Die wäßrige Phase wurde mit Natriumhydrugencarbanadösung (15 ml) auf pH 7-8 gebracht und nochmals mit Ethylacetat extrahiert (4 x 10 ml). Nach dem Trocknen der organischen Phase und Abdestillieren des Lösungsmittels wurden 465 mg (60 %) eines farblosen Feststoffes mit einem Schmelzpunkt bei 60-62°C erhalten.

**Beispiel 12:**

**Synthese von 5-Methyl-3-{[3'-(N,N-dimethylaminomethyl)-4'-hydroxy-4'-(3''-methoxyphenyl)-cyclohexylamino]-methyl}-1*H*-indol-2-carbonsäure**

5-Methyl- 3- { [3'-(N, N-dimethylaminomethyl)- 4'-hydroxy- 4'-(3"-methoxyphonyl) -cyclohexylamino] -methyl}-1*H*-indol-2-carbonsäuremethylester aus Beispiel 1 (800 mg. 1,62 mmul) wurde in Ethanol (10 ml) gelöst, mit 1N KOH (3,24 ml, 3,24 mmol) und Wasser (4 ml) versetzt und 10 h bei 60°C gerührt Ethanol wurde Im Vakuum abdestilliert und der Rückstand mit Wasser (10 ml) versetzt. Durch vorsichtige Zugabe von 1 N HCl (2 ml) wurde der pH-Wert der Mischung auf 7-8 eingestellt. Dle Konsistenz des Feststoffes veränderte sich dabei deutlich von schmierig zu kristallin. Der Feststoff wurde abgesaugt, mit Wasser gewaschen und im Vakuum über Phosphorpentoxid getrocknet Es wurde eine farblose Substanz (515 mg, 68%) mit einem Schmelzpunkt von 167-168°C erhalten.

**Beispiel 13:**

**Synthese von 5-Chlor-3-([3'-(N,N-dimethylaminomethyl)-4''-hydroxy-4'-(3"-methoxyphenyl)-cyclohexylamino] methyl}-1*H*-indol-2-carbonsäure**

Die Darstellung erfolgte aus 5-Chlor-3-{[3'-(N,N-dimethylaminomethyl)-4'-hydroxy. 4'-(3"-methoxyphenyl)-cyclohexylamino]-methyl}1*H*-indol-2-carbonsäureethylester analog zu Beispiel 12. Der Schmelzpunkt der Verbindung lag bel 232-234°C.

**Beispiel 14:**

**Synthese von 4,6-Dimethyl-3-{[3'-N,N-dimethylaminomethyl)4'-hydroxy-4'-(3"-methoxyphenyl)-cyclohexylamino]-methyl}-1*H*-Indol-2-carbonsäure,**

Die Darstellung erfolgte aus 4,6-Dimethyl-3-{[3'-(N,N-dimethylaminomethyl)-4'-hydroxy-4'-(3"-methoxyphenyl) -cyclohexylamino]-methyl}-1*H*-indol-2-carbonsäureethylester analog zu Beispiel 12. Der Schmelzpunkt der Verbindung lag bei 259-262°C.

**Beispiel 15:**

**Synthese von 4,6-Dichlor-3-([3'-(N.N-dimethylaminomethyl)-4"-hydroxy-4"-(3"-methoxyphenyl)-cyclohexyl-amlno]methyl}-1*H*-indol-Z-carbonsäure**

**Die Darstellung erfolgte aus 4,6-Dichlor-3-{[3'-(N,N-dimethylaminomethyl)4'-hydroxy-4'-(3"-methoxyphenyl)-cyclohexylamino]methyl}-1*H*-indol-2-**carbonsäureethylester analog zu Beispiel 12.

**Beispiel 16 (REFERENZ):**

**Synthese von 4,6-Dichlor-3-[3'-hydroxy-3'-(3''-methoxyphenyl)-piperidin-1'-ylmethyl]-1*H*-indol-2-carbonsäure**

Die Darstellung erfolgte aus 4,8-Dichlor-3-[3'-hyclroxy-3'-(3"-methaxyphenyl)-piperidin-1'-ylmethyl]-1*H*-indol-2-carbonsädreethylester analog zu Beispiel 12. Die Verbindung hatte zwei Schmelzbereiche bei 190-205°C und 240243°C.

**Beispiel 17 (REFERENZ):**

**Synthese von 5-Chlor-3'-[3'-hydroxy-3'-(3"-methoxyphenyl)-piperidin-1'-ylmethyl]-1*H*-indol-2-carbonsäure**

Die Darstellung erfolgte aus 5-Chlor-3-[3'-hydroxy-3'-(3"-methoxyphenyl)-piperidin-1'-ylmethyl]-1*H*-indol-2-carbonsäureethylester analog zu Beispiel 12. Die Verbindung hatte einen Schmelzbereich bei 189192°C.

**Beispiel 18 (REFERENZ)** :

**Synthese von 4,6-Dichlor-3-{[4'-hydroxy-4'-(3"-methoxyphenyl)-cyclohexylamino]-methyl}-1*H*-Indol-2-carbon-säureethylester**

Die Darstellung erfolgte analog zu Beispiel 1. 4,6-Dichlor-3-formyl-1H-indol-2-carbonsäureethylester wurde mit 4-Amino-1-(3'-methoxyphenyl)-cyclohexan-1-ol zur Reaktion gebracht Der Schmelzpunkt der Verbindung lag bei 138-140°C.

**Beispiel 19 (REFERENZ):**

**Synthese von 4,6-Dichlor-3-({[4'-hydroxy-4'-(3''-methoxyphenyl)-cyclohexyl]-(N-methylamlno)}-methyl)-1*H*-In-dol-2-carbonsäureethylester**

Die Darstellung erfolgte analog zu Beispiel 1.4,6Dichlor-3-formyl-1H-indol-2-carbonsäureethylester wurde mit 1-(3'-Methoxyphenyl)-4(N-methylamino)-cycrohexan-1-ol zur Reaktion gebracht Der Schmelzpunkt der Verbindung lag bei 55-57°C.

**Beispiel 20 (REFERENZ):**

**Synthese von 4,6-Dichlor-3-({[2'-hydroxy-2'-(3''-methoxyphenyl)-cyclohexylmethyl]-amino}-methyl)-1*H*-indol-2-carbonsäureethylester**

4,6-Dichlor-3-formyl-1H-indol-2-carbonsäureethylester (514 mg, 1,79 mmol) wurde in THF (20 ml) und 1,2-Dichlorethan (20 ml) gelöst, mit 2-(Aminomethyl)-1-(3'-methoxyphenyl)-cyclohexan-1-ol (423 mg, 1,78 mmol) und geglühtem Natriumsulfat (4 g) versetzt und 3 Tage bei Raumtemperatur gerührt. Es wurde Natriumtriacetoxyborhydrid (758 mg, 3,58 mmol) hinzugefügt und weltere 2 h gerührt. Zur Aufarbeitung wurde Natriumhydrogencarbonatlösung (20 ml) hinzugefügt, die Phasen getrennt, die wäßrige. Phase mit Diethylether (3 x 20 ml) und die vereinigten organischen Phasen mit Natriumhydrogencarbonatlösung (2 x 5 ml) extrahiert, die organischen Phase über Natriumsulfat getrocknet, das Lösungsmittel abdestilliert und 971 mg Rohprodukt erhalten. Das Rohprodukt wurde aus Ethylacetat/Ethanol (20 : 1, 15 ml) umkristallisiert Die Ausbaute betrug 749 mg (83 %). Die Verbindung fiel als farbloser Feststoff mit einem Schmelzpunkt bel 185-188 °C an.

**Beispiel 21** (**REFERENZ)** :

**Synthese von 5-Chlor-3-({[2'-hydroxy-2'-(3''-methoxyphenyl)-cyclohexylmethyl]amino}-methyl)-1*H*-indol-2-carbonsäureethylester**

5-Chlor-3-formyl-1H-indol-2-carbonsäureethytester (851 mg. 3,37 mmol) wurde in THF (20 ml) und 1,2-Dichlorethan (20 ml) gelöst mit 2-(Aminomethyl)-1-(3'-methoxyphenyl)-cyclohexan-1-ol (795 mg, 3.37 mmol) und geglühtem Natriumsulfat (3 g) versetzt und 48 h bei Raumtemperatur gerührt. Anschließend wurde Natriumtriacetoxyborhydrid (1 g. 4,7 mmol) hinzugefügt, weitere 6 h gerührt das Natriumsulfat abgesaugt, das lösungsmittel abdestiliert und der Rückstand In Ethylacetat (10 ml) gelöst Nach Zugabe von 1N Schwefelsäure (3 ml) und Wasser (10 ml) wurden die Phasen getrenat und die wäßige Phase mit Ethylacetat (8 x 10 ml) extrahiert. Die wäßrige Phase wurde mit Natriumhydrogencarbonat auf pH 8 gebracht und wieder mit Ethylacetat extrahiert (4x10 ml). Das Lösungsmittel wurde entfernt. Die Ausbeute betrug 740 mg (47 %). Der Schmelzpunkt der Verbindung lag bei 157-158 °C.

**Beispiel 22 (REFERENZ):**

**Synthese von 4,6-Dimethyl-3-({[2'-hydroxy-2'-(3''-methoxyphenyl)-cyclohexylmethyl]-amino}-methyl)-1*H*-indol-2-carbonsäureethylester**

4,6-Dimethyl-3-formyl-1H-indol-2-carbonsäureethylester (491 mg. 2 mmol) wurde in THF (20 ml) und 1.2-Dichlorethan (20 ml) gelöst, mit 2-(Aminomethyl)-1-(3'-methoxyphenyl)-cyclohexan-1-ol (471 mg, 2 mmol) und getrocknetem Natriumsulfat (4 g) versetzt und 48 h bei Raumtemperatur gerührt Nach Zugabe von Natriumtriacetoxyborhydrid (600 mg, 2,8 mmol) wurde weitere 3 h gerührt. Das Natriumsulfat wurde abgesaugt und das Lösungsmittelabdestilliert. Der Rückstand wurde aber Kieselgel chromatographiert (90 g Kieselgel, 900 ml Ethylacetat/Ethanol 20 :1) und lieferte 488 mg (50 %) eines farblosen Feststoffes mit einem Schmelzpunkt bei 114-116 °C.

**Beispiel 23 (REFERENZ):**

**Synthese von 5-Methyl-3-({[2'-hydroxy-2'-(3''.methoxyphonyl)-cyclohexylmethyl]-amino}-methyl)-1*H*-indol-2-carbonsäureethylester**

5-Methyl-3-formyl-1*H*-indol-2-carbonsäureethylester (462 mg, 2 mmol) wurde in THF (20 ml) und 1,2-Dichlorethan (20 ml) gelöst, mit2-(Aminomethyl)-1-(3'-methoxyphenyl)-cydohexan-1-ol (471 mg, 2 mmal) und geglühtem Natriumsulfat (4 g) versetzt und 48 h bei Raumtemperatur gerührt Nach Zugabe von Natriumtriacetoxyborhydrid (600 mg. 2,8 mmol) wurde weitere 3 h gerührt Das Natriumsulfat wurde abgesaugt und das Lösungsmittel abdestilliert. Der Rückstand wurde mit Natriumhydrogencarbonat und Diethylether versetzt, die wäßrige Phase mit Diethylether extrahiert (4 x 20 ml), der Extrakt getrocknet und eingeengt. Der Rückstand wurde mit Ethylacetat/Ethanol (10 ml, 20 : 1) behandelt. Dabei löste sich ein Teil der Substanz. Der ungelöste Teil war sauberer Ester (283 mg, 31 %). Aus dem gelösten Teil konnten weitere 218 mg (24 %) durch Chromatographie über Kleselgel gewonnen werden (90 g Kieselgel, 900 ml Ethylacetat/Ethanol, 20 : 1). Die Gesamtausbeute betrug 501 mg (55 %). Der farblose Feststoff hatte einen Schmelzpunkt bei 201-204 °C.

**Beispiel 24 (REFERENZ):**

**Synthese von 4,6-Dichlor-3-({[2'-hydroxy-2'-(3''-hydroxyphenyl)-cyclohexylmethyl]-amino}-methyl)-1*H*-indol-2-carbonsäureethylester-Hydrochlorid**

4,6-Dichlor-3-formyl-1H-indol-2-carbonsäureethylester (280 mg. 0,98 mmol) wurde in THF (20 ml) gelöst, mit 2-(Aminomethyl)-1-(3'-hydroxyphenyl)-cyclohexan-1-ol (217 mg, 0,98 mmol) und getrocknetem Natriumsulfat (2 g) versetzt und 48 h bei Raumtemperatur gerührt. Anschließend wurde Natriumtriacetoxyborhydrid (300 mg, 1,4 mmol) hinzugefügt und nochmals 3 h gerührt Das Natriumsulfat wurde abgesaugt, mit Ethylacetat (3 x 10 ml) gewaschen, das Filtrat mit 1 N HCl (5 ml) versetzt, die organische Phase mit 1N HCl (2 x 2 ml) extrahiert und die wäßrige Phase mit Ethylacetat (2 x 10 ml) extrahiert Die wäßrige Phase wurde mit Natriumhydrugencaeonatlösung (30 ml) auf pH 7 eingestellt und nochmals mit Ethylacetat (5 x 10 ml) extrahiert. Die organische Phase wurde getrocknet und das Lösungsmittel abdestilliert Es wurden 299 mg (62 %) eines Öls erhalten. Das Öl konnte durch Rühren (5 h) mit Trimethylchlorsian (0,112 ml, 0,885 mmol) in Methylethylketon (5 ml) in ein kristallines Hydrochlorid mit einem Schmelzpunkt bei 175-178 °C überführt werden.

**Beispiel 25** (**REFERENZ)** :

**Synthese von 5-Chlor-3-({[2'-hydroxy-2"-(3'"-hydroxyphenyl)-cyclohexylmethyl]amino}-mothyl)-1*H*-indol-2-carbonsäumethylester**

5-Chlor-3-formyl-1 H-indol-2-carbonsäumethylester (504 mg, 2 mmol) wurde in THF (20 ml) gelöst, mit 2-(Aminomethyl)-1-(3'-hydroxyphenyl)-cyclohexan-1-ol (443 mg, 2 mmol) und getrocknetem Natriumsulfat (2 g) versetzt und 48 h bei Raumtemperatur gerührt Anschließend wurde Natriumtriacetoxyborhydrid (600 mg, 2,8 mmol) hinzugefügt und nochmals 6 h gerührt Das Natriumsulfat wurde abgesaugt, das Lösungsmittel abdestilliert, der Rückstand in Ethylacetat (10 ml) gelöst, mit 10 %-iger Schwefelsäure (2 ml) und Wasser (10 ml) versetzt, die Phasen getrennt und die wäßrige Phasemit Ethylacetat extrahiert (5×10 ml). Die wäßrige Phase wurde mit Natriumhydrogencarbonatlösung (10 ml) auf pH 7 eingestellt und nochmals mit Ethylacetat (5x 10 ml) extrahiert Die organische Phase wurde getrocknet und das lösungsmittel abdestilliertDas erhaltene Rohprodukt wurde chromatographiert (20 g Kieselgel, 200 ml Ethylacetat/Ethanol, 20: 1) und lieferte 244 mg (26 %) eines farblosen Feststoffs mit einem Schmelzpunkt bei 88-90 °C.

**Beispiel 26 (REFERENZ):**

**Synthese von 4,6-Dimethyl-3-({[2'-hydroxy-2'-(3"-hydroxyphenyl)-cyclohexylmethyl]-amino}-methyl)-1*H*-indol-2-carbonsäurethylester**

4,6-Dichlor-3-formyl-1H-indol-2-carbonsäureethylester (485 mg, 1,98 mmol) wurde in THF (40 ml) gelöst 2-(Aminomathyl)-1-(3'-hydroxyphenyl)-cyclohexan-1-ol (438 mg, 1,98 mmol) und Natriumsulfat (4 g) wurden zugefügt und 72 h bei Raumtemperatur gerührt. Das Natriumsulfat wurde abgesaugt, das Lösungsmittel abdestilliert, der Rückstand in Ethylacetat (20 ml) gelöst, mit 10 %iger Schwefelsäure (3 ml) und Wasser (10 ml) versetzt. Es fiel ein Feststoff aus, der abgesaugt wurde. Die waßrlge Phase wurde mit Ethylacetat (3 x 10 ml) extrahiert, die wäßrige Phase mit Nabiumhydrogencarbonatlösung auf pH 7-8 gebracht und wieder mit Ethylacetat (4 x 10 ml) extrahiert. Die organische Phase wurde vom Lösungsmittel befreit Die Ausbeute betrug 720 mg (81 %). Der Schmelzpunkt der Verbindung lag bei 168-170°C.

**Beispiel 27 (REFERENZ) :**

**Synthese von 5-Methyl-3-({[2'-hydroxy-2'-(3"-hydroxyphenyl)-cyclohexylmethyl]-amino}-methyl)-1*H*-indol-2-carbonsäureethylester**

5-Methyl-3-formyl-1H-Indol-2-carbonsaureethylester (376 mg, 1,63 mmol) wurde in THF (20 ml) gelöst, 2-(Aminomethyl)-1-(3'-hydroxyphenyl)-cyclohexan-1-ol (360 mg, 1,63 mmol) und getrocknetem Natriumsulfat (3 g) versetzt und 48 h gerührt. Es wurde Natriumtriacetoxyborhydrid (482 mg, 2,28 mmol) hinzugefügt und weitere 4 h gerührt. Zur Aufarbeitung wurde das Natriumsulfat abgesaugt, das Lösungsmittel abdestilliert, der Rückstand mit Natriumhydrogencarbonatlösung (20 ml) und Ethylacetat (20 ml) versetzt, die Phase getrennt, die wäßrige Phase mit Ethylacetat (3 x 20 ml) extrahiert, die organische Phase über Natriumsulfat getrocknet und das Lösungsmittel abdestilliert Der Rückstand wurde chromatographiert (60 g Kieselgel, 600 ml Ethylacetat/Ethanol, 20 :1). Es konnten 360 mg (51 %) eines farblosen Feststoffes mit einem Schmelzpunkt bei 178-180 °C gewonnen werden.

**Beispiel 28 (REFERENZ):**

**Synthese von 4,6-Dichlor-3-({[2'-hydroxy-2'-(3''-methoxyphenyl)-cyclohexylmethyl]-amino}-methyl)-1*H*-indol-2-carbonsäure**

4,6-Dichlor-3-({[2'-hydroxy-2'-(3"-methoxyphenyl)-cyclohexylmethyl]amino}-methyl)-1*H*-indol-2-carbonsäureethylester aus Beispiel 23 (177 mg, 0,35 mmol) wurde In THF (5 ml) und Ethanol (10 ml) gelöst, mit 1 N KOH (0,7 ml, 0,7 mmol) und Wasser (2 ml) versetzt und 3,5 h bei 60°C gerührt. Zur Aufarbeitung wurde das Lösungsmittel abdestilllert, der halbfeste Rückstand mit Wasser (10 ml) versetzt und mit 1 N HCl (0,55 ml. 0,55 mmol) auf pH 7 eingestellt Der gebitlete Feststoff wurde abgesaugt, mit Wasser gewaschen und über $P_2O_5$getrocknet Die Ausbeute betrug 147 mg (88 %). Der Schmelzpunkt der Verbindung lag bei 244-245 °C.

**Beispiel 29 (REFERENZ) :**

**Synthese von 5-Chlor-3-({[2'-hydroxy-2'-(3"-methoxyphenyl)-cyclohexylmetlhyl]-amino}-methyl)-1*H*-indol-2-carbonsäure**

5-Chlor- 3-( { [2'-hydroxy- 2'-(3"-methoxyphenyl} -cyclohexylmethyl] -amino} methyl)- 1H-Indol- 2-carbonsäureethylester (360 mg, 0.764 mmol) wurde in Ethanol (12 ml) suspendiert, mit 1N KOH (1,53 ml, 1,53 mmol) versetzt und bel 60 °C 6 h gerührt. Nach Abdestillieren des Lösungsmittels wurde mit Wasser (10 ml) versetzt und mit 1 N HCl neutralisiert. Der gebildete Feststoff wurde abgesaugt und gatucknet Die Ausbeute betrug 290 mg (86 %). Der Schmelzpunkt der Verbindung lag bei 242-243 °C.

**Beispiel 30 (REFERENZ) :**

**Synthese von 4,6.Dimethyl-3-({[2'-hydroxy-2'-(3"-methoxyphenyl)-cyclohexylmethyl]-amino}-methyl)-1*H*-indol-2-carbonsäure**

4,6-Dimethyl-3-({[2'-hydroxy-2'-(3''-methoxyphenyl)-cyclohexylmethyl]amino)-methyl)-1*H*-indol-2-carbonsäureethylester (200 mg, 0,43 mmol) wurde in Ethanol (5 ml) gelöst, mit 1 N KOH (0,86 ml, 0,86 mmol) und Wasser (0,4 ml) versetzt und 5 h bei 60°C gerührt Nach dem Abdestillieren des Lösungsmittels. Zugabe von Wasser (10 ml) und Neutralisation mit 1N HCl wurde der gebildete Feststoff abgesaugt, gewaschen und getrocknet Die Ausbeute betrug 127 mg (68 %). Die Verbindung fiel als farbloser Feststoff mit einem Schmelzpunkt bel 210-213 °C an.

**Beispiel 31 (REFERENZ)** :

**Synthese von 5-Methyl-3-({[2'-hydroxy-2'(3"-methoxyphenyl)-cyclohexylmethyl]-amino}-methyl)-1*H*-indol-2-carbonsäure**

5-Methyl- 3-( { [2'-hydroxy- 2'-(3"-mathoxyphenyl) -cyclohexylmethyl] -amino} methyl)- 1*H*-indol- 2-carbonsäureethylester(250 mg. 0,55 mmol) wurde in Ethanol (10 ml) suspendiert, mit 1N KOH (1,1 ml, 1.10 mmol) und Wasser (2 ml) versetzt und 6 h bei 60 °C gerührt. Nach dem Abdestillieren des Lösungsmittels, Zugabe von Wasser (10 ml) und Neutralisation mit 1N HCl wurde der ausgefallene Feststoff abgesaugt, gewaschen und getrocknet Die Ausbeute betrug 209 mg (89 %). Der Schmelzpunkt der Verbindung lag bei 217-219 °C.

**Beispiel 32 (REFERENZ):**

**Synthese von 4,6-Dichlor-3-({[2'-hydroxy-2'-(3"-hydroxypheny)-cyclohexylmethyl]-amino}methyl)-1*H*-indol-2-carbonsäure**

4,6-Dichlor-3-({[2'-hydroxy-2'-(3"-hydroxyphenyl)-cyclohexylmethyl]-amino}-methyl)-1*H*-indol-2-carbonsäureethylester wurde in Ethanol suspendiert, mit 1N KOH und Wasser versetzt und 6 h bei 60 °C gerührt. Nach dem AbdestnUemn des Lösungsmittels, Zugabe von Wasser (10 ml) und Neutralisation mit 1N HCl wurde der ausgefallene Feststoff abgesaugt, gewaschen und getrocknet. Die Ausbeute betrug 201 mg (88 %). Der Schmelzpunkt der Verbindung lag bei 230232 °C.

**Beispiel 33 (REFERENZ):**

**Synthese von 5-Chlor-3-({[2'-hydroxy-2'-(3"-hydroxyphenyl)-cyclohexylmethyl]-amino}-methyl)-1*H*-Indol-2-carbonsäure**

5-Chlor-3-({[2'-hydroxy-2'-(3''-hydroxyphenyl)cyclohexylmethyl]-amino}-methyl)-1*H*-indol-2-carbonsäureethylester(90 mg, 0,197 mmol) wurde in Ethanöl (5 ml) gelöst, mit 1 N KOH (0,4 ml, 0,4 mmol) und Wasser (2 ml) versetzt und 6 h bei 60 °C gerührt Ethanol wurde abdestiliert, der Rückstand mit Wasser (10 ml) versetzt und mit 1 N HCl auf pH 7 eingestellt Der gebildete Feststoff wurde abgesaugt und getrocknet. Die Ausbeute betrug 63 mg (75 %). Der Schmelzpunkt der Verbindung lag bei 201-203 °C.

**Beispiel 34 (REFERENZ)**:

**Synthese von 4,6-Dichlor-3-[(1',5'.dimethyl-3'oxo-2'-phenyl-2',3'-dihydro-1'*H*-pyrazol-4',ylamino)-methyl]-1*H*-indol-2-carbonsäureethylester**

4,6-Dichlor-3-formyl-1*H*-indo-2-carbonsäureethylester (286 mg, 1.0 mol) und 4 Aminoantipyrin (203 mg, 1,0 mmol) wurden in trockenem Tetrahydrofuran (10 ml) gelöst, mit geglühtem Natriumsulfat (2 g) versetzt und 3 Tage gerührt. Anschließend wurde Natriumtriacetoxyborhydrid (300 mg, 1,4 mmol) und trockenes 1,2-Dichlorethan (10 ml) hinzugefügt und weitere 3 h gerührt. Zur Aufarbeitung wurde das Lösungsmittel im Vakuum abdestilliert, der Rückstand mit Ethylacetat (10 ml), Wasser (10 ml) und Natriumhydrogencarbonatlösung (10 ml) versetzt. Es fiel ein Feststoff aus, der abgesaugt wurde. Nach dem Trocknen wurden 370 mg (79 %) eines farblosen Feststoffes mit einem Schmelzpunkt bei 202-204 °C erhalten.

**Beispiel 35 (REFERENZ)**

**Synthese von 4,6-Dimethyl-3-[(1',5'-dimethyl-3'-oxo-2'-phenyl-2',3'-dihydro-1'*H*-pyrazol-4-ylamino)-methyl]-1*H*-indol-2-carbonsäureethylester**

4,6-Dimethyl-3-formyl-1H-indol-2-carbonsäureethylester (736 mg, 3,0 mol) und 4-Aminoantipyrin (610 mg, 3,0 mmol) wurden in trockenem Tetrahydrofuran (10 ml) gelöst, mit geglühtem Natriumsulfat (3 g) versetzt und 3 Tage gerührt Anschließend wurde Nablumtriacotoxyborhydrid (900 mg, 4,2 mmul) und trockenes 1,2-Dichlorethan (10 ml) hinzugefügt und weitere 3 h gerührt Zur Aufarbeitung wurde das Lösungsmittel im Vakuum abdestilliert, der Rückstand mit Ethylacetat (10 ml), Wasser (10 ml) und Natriumhydrogencarbonatlösung (10 ml) versetzt. Es fiel ein Feststoff aus, der abgesaugt wurde. Die Ausbeute betrug 384 mg (79 %). Der farblose Feststoff hatte einen Schmetzpunkt bel 200-202°C.

**Beispiel 36 (REFERENZ) :**

**Synthese von 4,6-Dichlor-3-[4'-(4"-chlorphenyl)-4'-hydroxypiperidin-1'-ylmethyl]-1*H*-indol-2-carbonsäureethylester**

4,6-Dichlor-3-formyl-1*H*-indol-2-carbonsäureethylester (858 mg, 3 mmol) und 4(4'-Chlorphenyl)-piperidin4-ol (635 mg, 3 mmol) wurden in trockenem Tetrahydrofuran (20 ml) gelöst, mit geglühtem Natriumsulfat (2 g) versetzt und 48 h bei Raumtemperatur gerührt. Anschließend wurden trockenes 1,2-Dichlorethan (10 ml) und Natriumtriacetoxyborhydrid (900 mg) hinzugefügt und weitere 3 h gerührt Zur Aufarbeitung wurde das Lösungsmittel im Vakuum abdestilliert und der Rückstand mit Wasser (10 ml), 1N Schwefelsäure (2 ml) und Ethylacetat (10 ml) versetzt. Der ausgefallenen Feststoff wurde abgesaugt mit Wasser gewaschen und getrocknet. Es wurden 1.13 g des Produkts erhalten. Der Schmelzpunkt der Verbindung lag bei 64-66°C.

**Beispiel 37 (REFERENZ):**

**Synthese von 4,6-Dimethyl-3-[4'-(4"-chlorphenyl)-4'-hydroxypiperidin-1'-ylmethyl]-1*H*-indol-2-carbonsäureethylester**

4,6-Dimethyl-3-formyl-1H-indol-2-carbonsäureethylester (1,47 g, 6 mmol) und 4(4'-Chlorphenyl)-piperidin-4-ol (1,27 g, 6 mmol) wurden in trockenem Tetrahydrofuran (30 ml) gelöst, mit geglühtem Natriumsulfat (4 g) versetzt und 24 h bei Raumtemperatur gerührt. Anschließend wurden trockenes 12-Dichlorethan (20 ml) und Natriumtriacetoxyborhydrid (1,78 g, 8,4 mmol) hinzugefügt und weitere 3 h bei Raumtemperatur gerührt Nach dem Abdestillieren des Lösungsmittels im Vakuum wurde der Rückstand mit Natriumhydrogencarbonatlösung (30 ml) und Ethylacetat (20 ml) versetrt,15 min. gerührt, die Phasen getrennt und die wäßrige Phase mit Ethylacetat extrahiert (3 x 20 ml). Die organische Phase wurde getrocknet und vom Lösungsmittel befreit Die Ausbeute betrug 1.54 g (58 %). Der Schmelzpunkt der Verbindung lag bei 65—67°C.

**Beispiel 38 (REFERENZ):**

**Synthese von 4,6-Dichlor-3-(6',7'-dimethoxy-3',4'-dihydro-1'*H*-isochinolin-2"-ylmethyl)-1*H*-indol-2-carbonsäureethyrester-Hydrochlorid**

6,7-Dimethoxy-3,4-dihydro-1H-isochlnolln (204 mg. 1.05 mmol) und 4,6-Dichlor-3-formyl-1H-indol-2-carbonsäureethylester (300 mg, 1,05 mmol) wurden in THF (10 ml) gelöst, nach 10 Minuten wurde die Lösung mit Natriumtriacetoxyborhydrid (300 mg, 1,4 mmol) versetzt und 22 h gerührt. Das Lösungsmittel wurde entfernt und der Rückstand mit Diethylether (20 ml) und 0,5N HCl (20 ml,10 mmol) versetzt und 1 h gerührt. Dabei kristallisierte das Hydrochlorid aus und konnte nach Waschen mit Diethylether und Wasser als weißer Feststoff mit einem Schmelzpunkt von 173-176 °C In einer Ausbeute von 83 % erhalten werden.

**Beispiel 39 (REFERENZ):**

**Synthese von 4,6-Dimethyl-3-(6',7'-dimethoxy-3',4'-dihydro-1'*H*-isochinolin-2'-ylmothyl)-1*H*-Indul-2-carbonsäureethylester-Hydrochlorid**

4,6-Dimethyl-3-formyl-1H-indol-2-carbonsäureethylester (637 mg, 2,6 mmol) und 6,7-Dimethoxy-3,4-dihydro-1H-isochinolin (510 mg, 2,6 mmol) wurden In Tetrahydrofuran (25 ml) gelöst nach 10 Minuten mit $NaBH(OAc)_3$ (780 mg, 364 mmol) versetzt und 72 h gerührt Das Reaktionsgemisch wurde eingeengt, der Rückstand mit Diethylether (40 ml) und 1N Salzsäure (26 ml, 26 mmol) versetzt und 1 h gerührt. Das ausgefallene Hydrochlorid wurde mit Diethylether und Wasser gewaschen. Das Produkt könnte als beigefarbener Feststoff mit einem Schmelzpunkt von 199-203 °C in reiner Form in einer Ausbeute von 76 % gewonnen werden.

**Beispiel 40 (REFERENZ) :**

**Synthese von 4,6-Dichlor-3-[4'-(3"-methoxyphenyl)-piperazin-1"-ylmethyl]-1H indol-2-carbonsäureethylester-Dihydrochlorid**

1-(3'-Methoxyphenyl)-piperazin (360 mg, 1,88 mmol) und 4,6-Dichlor-3-formyl-1H-indol-2-carbonsäureethylester (537 mg, 1,88 mmol) wurden in THF (18 ml) gelöst und nach 10 min mit $NaBH(OAc)_3$ (540 mg, 2.52 mmol) versetzt. Nach 22 h schien kein Umsatz: erfolgt zu sein, so daß nochmals $NaBH(OAc)_3$ (540 mg. 2.52 mmol) und wasserfreies Natriumsulfat (3.8 g) hinzugefügt wurde. Nach einer weiteren Reaktionszeit von 67 h wurde das Reaktionsgemisch eingeengt, der Rückstand in Diethylether (40 ml) und 0,5N HCl (50 ml, 25 mmol) aufgenommen und 1 h gerührt. Das ausgefallene Dihydrochlorid wurde abflltriert, mit Diethylether und Wasser gewaschen. Das Produkt konnte als beigefarbener Feststoff mit einem Schmelzpunkt von 169-172 °C in einer Ausbeute von 85% gewonnen werden.

**Beispiel 41 (REFERENZ):**

**Synthese von 4,6-Dichlor-3-(4'-carbamoyl-[1'',4]bipiperidin-1'-ylmethyl)-1*H*-indol-2-carbonsäureethylester**

[1,4]-Bipiperidinyl-4'-carbonsäureamid (423 mg, 2 mmol) und 4,6Dichlor-3-formyl-1 H-Indol-2-carbonsäureethylester. (572 mg, 2-mmol) wurden in THF (20 ml) gelöst ; nach 10 Minuten wurde $NaBH(OAc)_3$ (800 mg, 2,8 mmol) hinzugefügt und 24 h gerührt. Die Mischung wurde eingeengt, der Rückstand In Diethylether (40 ml) und in 0,5N Salzsäure (40 ml, 20 mmol) aufgenommen und 2 h gerührt, wobei das Dihydrochlorid als Rohprodukt ausfiel. Der Feststoff wurde abgesaugt und mit Diethylether gewaschen. Eine weltere Reinigung mit Wasser war wegen der guten Löslichkeit des Dihydrochlorids nicht möglich. Das Dihydrochlorid wurde in Ethanol (60 ml) aufgenommen, wobei keine Klare Lösung enstand, mit 5 %iger $NaHCO_3$-Lösung (30 ml) versetzt und 1 h gerührt. Ein weißer Feststoff wurde abgesaugt und mit Ethanol und Wasser gewaschen. Das Produkt wurde in einer Ausbeute von 44 % gewonnen und hatte einem Schmelzpunkt von 212-215 °C.

**Beispiel 42 (REFERENZ):**

**Synthese von 4,6-Dichlor-3-{[(4'-benzyl-4' -(N,N-dimethylamino)-cyclohexyl)-(N-propylamino)]-methyl}-1*H*-indol-2-carbonsäureethylester-Dihydrochlorid**

4,6-Dichlor-3-formyl-1H-indol-2-carbonsäureethylester (519 mg, 1,81 mmol) und 1-Benzyl-1-(N,N-dimethylamino)-4-(N-propylamino)-cyclohexan (500 mg, 1,81 mmol) wurden unter Argon in THF (18 ml) gelöst und mit $Na_2SO_4$

(3,62 g) versetzt. Nach 15 Minuten wurde $NaBH(OAc)_3$ als Reduktionsmittel hinzugefügt und die Mischung 23 h gerührt. Das Lösungsmittel wurde entfernt, der Rückstand in Diethylether (40 ml) und 1N HCl (18 ml, 18 mmol) aufgenommen und 2 h gerührt. Erst nach 3 Tagen ließ sich das Hydrochlorid als Feststoff isolieren. Der Feststoff wurde abfiltiert und mit Diethylether gewaschen. Zur weiteren Reinigung wurde das Hydrochlorid in Ethylacetat (60 ml) aufgenommen, mit 5 %iger $NaHCO_3$-Lösung (20 ml) versetzt und gerührt Nach dem Einengen wurde die freie Base in einer Ausbeute von 49 % erhalten. Das Dihydrochlorid konnte durch Aufnahme der Base in Ethylmethylketon (15 ml) und Zugabe von $ClSiMe_8$ (0,132 ml, 1,05 mmol) nach 1 h Reaktionszeit als weißer Feststoff mit einem Schmelzpunkt von 165-169 °C in einer Ausbeute von 39 % gewonnen werden.

**Belspiel 43 (REFERENZ) :**

**Synthese von 4,6-Dichlor-3[(1',5'-dimethyl-3'-oxo 2'-phenyl-2',3'-dihydro-1'*H*-pyrazol-4"-ylamino)-methyl]-1*H*-indol-2-carbonsäure**

4,6-Dichlor-3-[(1', 5'-dimethyl-3'-oxo-2'-phenyl-2', 3'-dihydro-1'*H*-pyrazol-4-ylamino)-methyl]-1*H*-indol-2-carbonsäureethylester (370 mg, 0,781 mmol) wurde in Ethanol (10 ml) gelöst, mit 1N KOH (2.6 ml, 2,6 mmol) und Wasser (2 ml) versetzt und 3 h bei 60 °C gerührt Das Lösungsmittel wurde entfernt, der Rückstand mit Wasser (10 ml) versetzt und mit 1N HCl pH 8 eingestellt. Der gebildeten Feststoff wurde abgesaugt und mit Wasser gewaschen. Nach Trocknen im Vakuum wurden 233 mg (67 %) des Produkts gewonnen. Das Produkt hatte einen Schmelzpunkt von. 265-268 °C.

**Beispiel 44 (REFERENZ)**:

**Synthese von 4,6-Dichlor-3-[4'-(4"-chlorphenyl)-4'-hydroxypiperidin-1'-ytmethyl]-1*H*-indol-2-carbonsäure**

4.6-Dichlor-3-[4'-(4"-chlorphenyl)-4'-hydroxypiperidin-1'-ylmethyl]-1*H*-indol-2-carbonsäureethylester (300 mg. 0,62 mmol) wurde in Ethanol (10 ml) gelöst und mit 1N KOH (1,25 ml. 1,25 mmol) und Wasser (2 ml) versetzt. Die Mischung wurde 4 h bel 60 °C gerührt, wobei sie sich rosa färbte. Das Lösungsmittel wurde abdestilliert, der Rückstand mit Wasser (10 ml) versetzt und mit 1 N HCl auf pH 6 eingestellt Es konnten 193 mg (68 %) eines leicht rosa gefärbten Feststoffes mit einem Schmelzpunkt von 170-174 ° C absaugen werden.

**Beispiel 45 REFERENZ :**

**Synthese von 4,6-Dimethyl-3-[4'-(4''-chlorphenyl)-4'-hydroxypiperidin-1'-ylmethyl]-1*H*-indol-2-carbonsäure**

4,6-Dimethyl-3-[4'-(4''-chlorphenyl)-4'-hydroxypiperidin-1'-ylmethyl]-1*H*-indol-2-carbonsaureethylester (300 mg. 0,68 mmol) wurde In Ethanol (10 ml) gelöst, mit 1 N KOH (2.36 ml, 2.36 mmol) und Wasser (1 ml) versetzt und 4 h bei 60 °C gerohrt. Die Mischung färbte sich rot Zur Aufarbeitung wurde das Lösungsmittel im Vakuum abdestilliert, der Rückstand mit Wasser (10 ml) versetzt und mit 1N HCl auf pH 6 eingestellt Es konnten 190 mg (68 %) eines gelben Feststoffes mit einem Schmelzpunkt bei 194-197 °C gewonnen werden.

**Beispiel 46 (REFERENZ):**

**Synthese von 4,6-Dichlor-3-[4'-(3"-methoxyphenyl)-piperazin-1'-ylmethyl]-1*H*-indol-2-carbonsäure-Dihydrochlorid**

4,6-Dichlor-3-[4'-(3"-methaxyphenyl)-piperazin-1'-ylmethyl]-1*H*-indol-2-carbonsäureethylester-Dihydrochlorid (485 mg, 0,9 mmol) wurde in Ethanol (60 ml) gelöst und zur Hydrolyse mit 1,7N KOH (16 ml, 27 mmol) versetzt. Nach 16 Stunden Reaktionszeit wurde die Mischung eingeengt, das Kaliumsalz von 4,6 Dichlor-3-[4'-(3"-methoxyphenyl) -piperazin-1'-ylmethyl]-1*H*-indol-2-carbibsäure mit Ethylacetat extrahiert und durch Zugabe von 1,8N ethanolischer HCl (3 ml, 5,4 **mmol) das Dihydrochlorid von 4,6-Dichlor-3-[4'-(3''-methoxyphenyl)-piperazin-1'-**ylmethyl]-1*H*-indol-2-carbonsäure gebildet das ausfiel. Der Feststoff wurde abgesaugt, das Filtrat wurde weiter eingeengt und der Rückstand mit Wasser (10 ml) gewaschen. Das Produkt fiel als grauer Feststoff mit einem Schmelzpunkt von 177-182 °C in einer Ausbeute von 86 % an.

**Beispiel 47 (REFERENZ):**

**Synthese von 4,6-Dichlor-3-(6',7'-dimethoxy-3',4'-dihydro-1'*H*-isochinolin-2'-ylmethyl)-1*H*-indol-2-carbonsäure-Hlydrochlorid**

4,6-Dichlor- 3-(6', 7'-dimethoxy- 3', 4'-dihydro- 1*H*-isochinolin- 2'-ylmethyl)- 1*H*4Indol- 2-carbonsäureethylester (460 mg, 0,92 mmol) wurde in Ethanol (20 ml) gelöst, mit 1.7N KOH (11,4 ml, 20 mmol) versetzt und 20 h umgesetzt. Nach dem Einengen der Reaktionsmischung wurde der Rückstand mit Ethylacetat versetzt, wobei das Kaliumsalz von 4,6-Dichlor-3-(6',7'-dimethoxy-3',4'-dihydro-1'*H*-isochinolin-2'-ylmethyl)-1*H*-Indol-2-carbonsäure nach einige Minuten ausfiel. Das Kaliumsalz wurde abfiltriert, das Filtrat eingeengt und nochmals filtiert. Der Feststoff wurde in Ethylmethylketon (15 ml) gelöst und mit 1,8N ethanolischer HCl (1,15 ml, 2,08 mmol) versetzt Nach dem Abkühlen auf-10 °C wurde die Lösung eingeengt. Es fiel das Hydrochlorid von 4,6-Dichlor-3-(6',7-dimethoxy-3',4'-dihydro-1'*H*-isochinolin-2'-ylmethyl)-1*H*-indol-2-carbonsäure gemeinsam mit KCl aus und wurde abfiltiert. Der Feststoff wurde mit Wasser (5 ml) gewaschen. Das Produkt wurde mit einer Ausbeute von 80 % gewonnen. Der beigefarbene Feststoff hatte einen Schmelzpunkt von 186-192 °C.

**Beispiel 48 (REFERENZ):**

**Synthese von 4,6-Dimethyl-3-(6',7'-dimethoxy-3',4'-dihydro-1'*H*-isochinolin-2-ylmethyl)-1 *H*-indol-2-carbonsäure-Hydrochlorid**

4,6-Dimethyl- 3-(6', 7'-dimethoxy- 3', 4'-dihydro- 1*H*-isochinolin- 2'-ylmethyl)- 1*H*-indol- 2-carbonsäureethylester (300 mg, 0,71 mmol) wurde in Ethanol (60 ml) gelöst, mit 1,7N KOH (4.17 ml, 7,1 mmol) versetzt und 88 h umgesetzt. Nach dem Einengen der Mischung wurde das entstandene Kaliumsalz von 4,6Dimethyl-3-(6',7'-dimethoxy-3',4'-dihydro-1'*H*-isochinolin-2'-ylmethyl)-1*H*-indol-2-carbonsäure mit Ethylacetat und 1,8N ethanolischer HCl (1,56 ml. 1,42 mmol) versetzt. Nach dem Einengen auf 20 ml fiel das Hydrochlorid von 4,6-Dimethyl-3(6',7'-dimethoxy-3',4'-dihydro-1'*H*-isochinolin-2'-ylmethyl)-1*H*-indol-2-carbonsäure aus. Der Feststoff wurde abgetrennt und mit Wasser (10 ml) gewaschen. Das Produkt konnte mit einer Ausbeute von 61 % gewonnen werden. Der graue Feststoff hatte einen Schmelzpunkt von 176-180 °C.

**Beispiel 49 (REFERENCE):**

**Synthese von 4,6.Dichlor-3-[4'-(3"-methoxyphenyl)-piperazin-1'-ylmethyl]·1*H*-indol-2-carbonsäure-Dihydrochlorid**

4.6-Dichlor-3-[4'-(3"-methoxyphenyl)-piperazin-1'-ylmethyl]-1*H*-indol-2-carbonsäureethylester-Dihydrochlorid (485 mg, 0,9 mmol) wurde in Ethanol (60 ml) gelöst und mit 1,7N KOH (16 ml. 27 mmol) versetzt. Nach 16 Stunden Reakdonszeit wurde die Mischung eingeengt Das Kaliumsalz von 4,6-Dichlor-3 [4'-(3"-methoxyphenyl)-piperazin-1'-ylmethyl]-1*H*-indol-2-carbonsäure wurde mit Ethylacetat und 1,8N ethanolischer HCl (3 ml, 5,4 mmol) versetzt Das gebildete Dihydrochlorid wurde abgesaugt, das Filtrat weiter eingeengt und der Rückstand mit Wasser (10 ml) gewaschen. Das Produkt wurde mit einer Ausbeute von 86 % erhalten. Der graue Feststoff hatte einen Schmelzpunkt von 177-482 °C.

**Beispiel 50 (REFERENZ) :**

**Synthese von 4,6-Dichlor-3-(4'carbamoyl-[1",4]bipiperidin-1'-ylmethyl)-1*H*-indol-2-carbonsäure-Kaliumsalz**

4,6-Dichlor-3-(4'-carbamoyl-[1",4']bipiperidin-1'-ylmethyl)-1*H*-indol-2-carbonsäureethylester (476 mg, 0,98 mmol) wurde in Ethanol (90ml) gelöst mit 1,7N KOH (17,6 ml, 30 mmol) versetzt und 14 h gerührt Beim Einengen des Ansatzes schied sich ein farbloses Öl ab, das nach einlgen Minuten fest wurde, abgesaugt und mit Wasser gewaschen wurde. Das Kaliumsalz von 4,6-Dichlor-3-(4'carbamoyl-[1",4']bipiperidin-1'-ylmethyl)-1*H*-indol-2-carbonsäure fiel als grauer Feststoff mit einem Schmelzpunkt von 193-196 °C in einer Ausbeute von 70 % an-Eine verseifung der Amidfunktion konnte nicht beobachtet werden.

**Beispiel 51:**

**Synthese von 1-*tert*-Butoxycarbonyl-4,6-dichlor-3-{[3'-(N,N-dimethylaminomethyl)-4'-hydroxy-4'-(3''-methoxyphenyl)-cyclohexylamino]-methyl}-indol-2-carbonsäureethylester**

1-*tert*-Butoxycarbonyl-4,6-dichlor-3-(brommethyl)-indol-2-carbonsäursethylester und 4-Amino-2-(N,N-dimethylaminomethyl)-1-(3'-methoxyphenyl)-cyclohexan-1-ol wurden in Gegenwart von Caesiuncarbonat in DMF umgesetzt Nach einer Reaktionszeit von 18 h bei Raumtemperatur fiel das Produkt in einer Ausbeute von 55 % an. Die Reinigung erfolgte durch Flashchromatographie.

**Beispiel 52:**

**Synthese von 4,6-Dichlor-3-{[3'-(N,N-dimethylaminomethyl)-4'-hydroxy-4'-(3"-methoxyphenyl)-cyctohexylamino]methyl}-1-methyl-indol-2-carbonsäureethylester.**

4,6-Dichlor-3-formyl-1-methyl-indol-2-carbonsäureethylester und 4-Amino2-(N,N-dimethylaminomethyl)-1-(3'-methoxyphenyl)-cyclohexan-1-ol wurden in Gegenwart von Molsieb 4A in Diethylether in das Imin überführt. Das Imin wurde nach Entfernen von Molsleb und Lösungsmittel in Ethanol mit Natriumcyanoborhydrid zum Amin reduziert. Die Reinigung erfolgte durch Chromatographie. Das Produkt wurde in einer Ausbeute von 45 % erhalten.

**Beispiel 53 (REFERENZ):**

**Synthese von 4,6-Dichlor-3-[4'-(4"-chlor-3-trifluormethylphenyl)-4'-hydroxypiperidin-1-ylmethyl]-1*H*-indol-2-carbonsäureethylester**

4,6-Dichlor-3-formyl-1*H*-indol-2-carbonsäureethylester (286 mg, 1 mmol) wurde in THF (10 ml) gelöst und mit 4'-(4"-Chlor-3"-trifluomethylphenyl)4'-hydroxypiperidin (279 mg, 1 mmol) versetzt. Nach 10 Minuten wurde Natriumtriacetoxyborhydrid (300 mg, 1,4 mmol) hinzugegeben und anschließend 18 h gerührt Das Reaktionsgemisch wurde filtriert, der Rückstand mit Ethylacetat (60 ml) und 5% iger $NaHCO_3$ (15 ml) versetzt und die Mischung 15 Minuten gerührt. Die Phasen wurden getrennt und die organische Phase mit Wasser ausgeschüttelt, getrocknet und eingeengt. Es wurde ein weißer Feststoff mit einem Schmetzpunkt von 89-91 °C in einer Ausbeute von 55 % gewonnen.

**Beispiel 54 (REFERENZ):**

**Synthese von 4,6-Dimethyl-3-[4-(4"-chlor-3".trifluormethylphenyl)-4'-hydroxypipendin-1'-yimethyl]-1*H* indol-2-carbonsäurethylester**

4.6-Dimethyl-3-formyl-1*H*-indol-2-carbonsäurethytester (490 mg, 2 mmol) und 4'-(4"-Chlor-3"-trifluonnethylphenyl)-4=hydroxpiperidin (559 mg, 2 mmol) wurden in Tetrahydrofuran (20 ml) gelöst. Nach 10 Minuten wurde NaBH (OAc) (600 mg, 2,8 mmol) hinzugefügt. Nach einer Reaktionszeit von 19 h wurde die Mischung eingeengt, der Rückstand mit Diethylether (40 ml) und 0.5N HCI (40 mi, 20 mmol) versetzt und die Mischung 1 h genährt wobei das Hydrochlorid von 4.6-Dimethyl-3-[4'-(4".chlor-3"-trifluormethylphenyl)-4'-hydroxypipendin-1'-yhnethyl]-1*H*-indol-2-carbonsäureethylester als Feststoff ausfiel. Der Feststoff wurde abgesaugt und anschließend mit Diethylether und Wasser gewaschen. Das Rchprodukt wurde mit Ethylacetat (20 ml) und 5%-iger $NaHCO_3$ (20 ml) versetzt und die Mischung 20 min gerührt. Die Phasen wurden getrennt und die organische Phase eingeengt Es wurde ein der beiger Feststoff mit einem Schmelzpunkt von 77-81 °C in einer Ausbeute von 60 % gewonnen.

**Beispiel 55 (REFERENZ):**

**Synthese von 4,6-Dichlor-3-[4'-(4"-chlor-3"-trifluormethylphzenyl)-4'-hydroxypiperidin-1'-ylmethyl]-1*H*-indol-2-carbonsäure-Hydrochlorid**

4,6-Dichlor- 3-[4'-(4"-chlor- 3"trifluormethyl) phenyl- 4'-hydroxypiperidin- 1'-ylmethyl]- 1*H*-Indol- 2-carbonsäurethylester (550 mg, 1 mmol) wurde In Ethanol (30 ml) aufgenommen, mit 1,7N KOH (15 ml, 25,5 mmol) versetzt und 16 h gerührt. Nach dem Einengen des Reaktionsgemisches wurde das Kaliumsalz von 4,6 Dichlor-3-[4'-(4"-chlor-3"-trifluomethylphenyl)-4'-hydroxypiperidin-1'-ylmethyl]-1*H*-indol-2 carbonsäure aus dem wäßrigen Rückstand mit Ethylacetat extrahiert und anschließend die organische Phase mit 1,8N ethanolischer Salzsäure (1,66 ml, 3 mmol) versetzt. Die Lösung wurde eingeengt und der Rückstand mit Wasser (2 x 10 ml) gewaschen. Das Produkt wurde in einer Ausbeute

von 66 % als weißes kristallines Produkt mit einem Schmelzpunkt von 206-209 °C gewonnen.

**Beispiel 56 (REFERENZ):**

**Synthese von 4,6-Dimethyl-3-[4'-(4"-chlor-3"-trifluormethylphenyl)-'4-hydroxypiperidin-1'-ylmethyl]-1*H*-indol-carbonsäure-Hydrochlorid**

Zu einer Lösung von 4,6-Dimethyl-3-[4'-(4"-chlor-3"-trifluormethylphenyl)-4'-hydroxypiperidin-1'-ylmethyl]-1*H*-indol-2-carbonsäure (332 mg, 0,65 mmol) in Ethanol (20 ml) wurde 1,7N KOH (7,6 ml, 13 mmol) gegeben und die Mischung 16 Stunden gerührt Nach dem Einengen der Lösung wurde mit Ethylacetat das Kaliumsalz der 4,6-Dimethyl-3-[4'-(4-chlor-3"-trifluormethylphenyl)-4'-hydroxypiperidin-1'-ylmethyl]-1*H*-indol-2-carbonsäure extrahiert und der Extrakt mit 1,8N ethanolischer Satzsäure (2 ml, 3,6 mmol) versetzt. Beim Einengen der Lösung fiel 4,6-Dimethyl-3-[4'-(4"-chlor-3"-trifluomethylphenyl)-4'-hydroxypiperidin-1'-ylmethyl]-1*H*-indol-2-carbonsäure-Hydrochlorid zusammen mit Kaliumchlorid aus. Durch Waschen mit Wasser konnte sauberes Hydrochlorid mit einer Ausbeute von 89 % erhalten werden. Das Produkt fiel als grauer Feststoff mit einem Schmelzpunkt von 184-188 °C an.

**Pharmakologische Untersuchungen**

**Analgesieprüfung im WrithingTest an der Maus**

Die vertiefte Untersuchung der erfindungsgemäßen Verbindungen auf analgetische Wirksamkeit wurde nach obenstehend beschriebenen Phenylchinoninduzierten Writhing an der Maus durchgeführt. Alle untersuchten erfindungsgemäßen Verbindungen zeigten eine mittelstarke bis starke analgetische Wirkung.

Die untersuchten erfindungsgemäßen Verbindungen zeigten eine analgetische Wirkung. Die Ergebnisse ausgewählter Writhing-Untersuchungen sind in der nachfolgenden Tabelle 1 zusammengefaßt.

Tabelle1 :

| Verbindung Nr. | % Hemmung der Writhing Reaktionen 10 ms/kg i.v. |
|---|---|
| 1 | 48 |
| 2 | 97 |
| 3 | 72 |
| 4 | 100 |

**Verbindungen Nr. 1-4**

1: 5-Chlor-3-{[3-(N,N-dimethylaminomethyl)-4'-hydroxy-4'-(3"-methoxyphenyl)-cyclohexylamino]-methyl}-1*H*-indol-2-carbonsäureethytester.

2: 4,6-Dichlor-3-{[3'-(N,N-dimethylaminomethyl)-4'-hydroxy-4'-(3"-methoxyphenyl)-cydohexylamino]-methyl}-1*H*-indol-2-carbonsäureethylester,

3: 4,6-Dichlor-3-{[3'-(N,N-dimethylaminomethyl)-4'-hydroxy-4'-(3"-methoxyphenyl)-cyclohexylamino]-methyl}-1*H*-indol-2-carbonsäure,

4: 1-tert-Butoxycarbonyl-4,6-dichlor-3-{[3'-(N,N-dimethylaminomethyl)-4'-hydroxy-4'-(3"-methoxyphenyl)-cyclohexylamino]methyl}-indol-2-carbonsäureethylester.

## Patentansprüche

1. Substituierte Indole der allgemeinen Formel I,

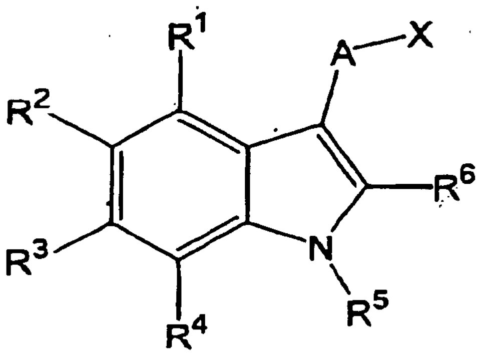

I

worin
$R^1$, $R^2$, $R^3$ und $R^4$, gleich oder verschieden, für einen linearen oder verzweigten, gesättigten oder ungesättigten aliphatischen $C_{1-10}$-Rest oder einen gesättigten oder ungesättigten cycloaliphatischen $C_{3-7}$Rest, wobei jeder der vorstehend genannten Reste gegebenenfalls über eine Etherbrücke gebunden sein kann, oder Wasserstoff, ein Halogen oder eine Hydroxygruppe stehen,
$R^5$ für Wasserstoff, einen linearen oder verzweigten, gesättigten oder ungesättigten aliphatischen $C_{1-10}$-Rest oder einen gesättigten oder ungesättigten cycloaliphatischen $C_{3-7}$-Rest, einen Aryl- oder Heteroarylrest, wobei der Aryl- oder Heteroarylrest gegebenenfalls über eine $C_{1-6-}$Alkylengruppe gebunden sein kann, einen substituierten Sulfonylrest oder eine Gruppe der Formel $-COR^7$ steht, wobei $R^7$ die unten angegebene Bedeutung hat,
$R^8$ für eine Gruppe der Formel $-COR^7$ steht, wobei $R^7$ die nachstehende Bedeutung hat,
$R^7$ für die Gruppe $OR^8$ steht, wobei der Rest $R^8$ die nachstehende Bedeutung hat,
$R^8$ für Wasserstoff oder für einen linearen oder verzweigten, gesättigten oder ungesättigten aliphatischen $C_{1-10}$-Rest steht,
A für eine Brücke der Formel $-(CH_2)_2-$ oder $-(CH_2)_nNR^{1'}-$, steht, worin n für 0. 1,2 oder 3 steht, $R^{1'}$ die nachstehend angegebene Bedeutung hat und die Bindung zum Rest X immer zuletzt genannt ist,
und X für den folgenden Rest steht, worin der freie Strich die Bindung zur Brücke A symbolisiert

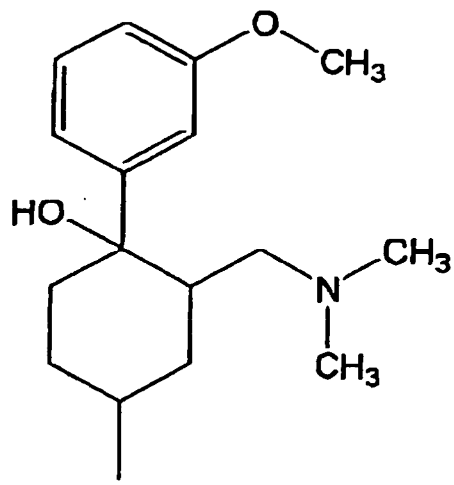

und
$R^1$ für Wasserstoff oder für einen linearen oder verzweigten, gesättigten oder ungesättigten aüphatischen $C_{1-10}$-Rest steht,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

2. Substituierte Indole gemäß Anspruch 1, **dadurch gekennzeichnet, daß** $R^2$ für einen linearen oder verzweigten,

gesättigten oder ungesättigten aliphatischen $C_{1-3}$-Rest oder ein Halogen und $R^1$, $R^3$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

3. Substituierte Indole gemäß Anspruch 1, **dadurch gekennzeichnet, daß** $R^3$ für einen linearen oder verzweigten, gesättigten oder ungesättigten aliphatischen $C_{1-3}$-Rest oder ein Halogen und $R^1$, $R^2$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

4. Substituierte Indole gemäß Anspruch 1, **dadurch gekennzeichnet, daß** $R^2$ und $R^3$, gleich oder verschieden, für einen linearen oder verzweigten, gesättigten oder ungesättigten aliphatischen $C_{1-3}$-Rest oder ein Halogen und $R^1$ und $R^4$ jeweils für Wasserstoff stehen, gegebenenfalls in Form ihrer Racemate, ihrer reinen Stereoisomeren, Insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

5. Substitulerte Indole gemäß Anspruch 1, **dadurch gekennzeichnet, daß** $R^1$ und $R^3$, gleich oder verschieden, für einen linearen oder verzweigten, gesättigten oder ungesättigten aliphatischen $C_{1-3}$-Rest oder ein Halogen und $R^2$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

6. Substituierte Indole gemäß Anspruch 1, **dadurch gekennzeichnet, daß** $R^2$ für einen Methylrest oder ein Chlor und $R^1$, $R^3$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

7. Substituierte Indole gemäß Anspruch 1, **dadurch gekennzeichnet, daß** $R^3$ für einen Methylrest oder ein Chlor und $R^1$, $R^2$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

8. Substituierte Indole gemäß Anspruch 1, **dadurch gekennzeichnet, daß** $R^2$ und $R^3$ jeweils für einen Methylrest oder ein Chlor und $R^1$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, Insbesondere der physiologisch verträglichen Salze, oder in Form ihrer Solvate, insbesondere der Hydrate.

9. Substituierte Indole gemäß Anspruch 1, **dadurch gekennzeichnet, daß** R' und $R^3$ jeweils für einen Methylrest oder ein Chlor und $R^2$ und $R^4$ jeweils für Wasserstoff stehen, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

**10.** Substituierte Indole gemäß Anspruch 1, **dadurch gekennzeichnet, daß** $R^5$ für Wasserstoff steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

**11.** Substituierte Indole gemäß Anspruch 1, **dadurch gekennzeichnet, daß** $R^6$ für eine Gruppe der Formel $COR^7$ steht, wobei $R^7$ für die Gruppe $OR^8$ und der Rest $R^8$ für Wasserstoff oder einen linearen oder verzweigten, gesättigten oder ungesättigten aliphatischen $C_{1-3}$-Rest, vorzugsweise eine Methyl- oder Ethylgruppe, steht, gegebenenfalls in Form ihrer Racemate, ihrer reinen Stereoisomeren, insbesondere Enantiomeren oder Diastereomeren, oder in Form von Mischungen der Stereoisomeren, Insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder In Form Ihrer Solvate, insbesondere der Hydrate.

**12.** Substituierte Indole gemäß Anspruch 1, **dadurch gekennzeichnet, daß** A für eine Brücke mit einer der folgenden Formeln steht: $-CH_2-$, $-CH_2NR^{1'}-$, worin $R^{1'}$ für Wasserstoff oder eine lineare oder verzweigte, gesättigte oder ungesättigte aliphatische $C_{1-3}$-Gruppe steht, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer Salze, insbesondere der physiologisch verträglichen Salze, oder in Form ihrer Solvate, insbesondere der Hydrate.

**13.** Ein substituiertes Indol gemäß Anspruch 1 ausgewählt aus der Gruppe umfassend:

5-Methyl- 3- { [3'-(N, N-dimethylaminomethyl)- 4'-hydrvxy- 4'-(3"-methoxyphenyl) -cyclohexylamino] -methyl}-1*H*-indol-2-carbonsäuremeihylester,
4,6-Dimethyl-3-{[3'-(N,N-dimethylamlnornethyl)-4'-hydroxy-4'-(3"-methoxyphenyl)-cyclohexylamino]-methyl}-1*H* indol-2-carbonsäureethylester,
5-Chlor-3-[3'-(N,N-dimethylaminomethyl)-4'-hydroxy-4'-(3"-methoxyphenyl)-cyclohexylamino]-methyl}-1*H*-indol-2-carbonsäunrethylester,
4,6-Dichlor- 3- { [3'-(N, N-dimethytaminomethyl)- 4'-hydroxy- 4'-(3"-methoxyphenyl) -cyclohexylamino] -methyl}. 1*H*-indol-2-carbonsäureethylester,
4,6-Dimethyl- 3- { [3'-(N, N-dimethylaminomethyl)- 4'-hydroxy- 4'-(3"-methoxyphenyl) -cyclohexyl-(N-methylamino)]-methyl}-1*H*-indol-2-carbonsäureethylester.
4,6-Dichlor-3-{[3'-(N,N-dimethylaminomethyl)-4'-hydroxy-4'-(3"-methoxyphenyl)-cyclohexyl-(N-methylamino)] -methyl}-1*H*-indol-2-carbonsäureethylester,
5-Chlor- 3- { [3'-(N, N-dimethylaminomethyl)- 4'-hydroxy- 4'-(3"-methoxyphenyl) -cyclohexyl-(N-methylamino)] -methyl}-1*H*-indol-2-carbonsäureethylester,
5-Methyl- 3- { [3'-(N, N-dimethylaminomethyl)- 4'-hydroxy- 4'-(3"-methoxyphenyl) -cyclohexylamino] -methyl}- 1 *H*-indol-2-carbonsäure,
5-Chlor-3-{[3'-(N,N-dimethylaminomethyl)-4'-hydroxy-4'-(3"-methoxyphenyl)-cyclohexylamino]-methyl}-1 *H*-indol-2-carbonsäure,
4,6-Dimethyl- 3- { [3'-(N, N-dimethylaminomethyl)- 4'-hydroxy- 4-(3"-methoxyphenyl) -cydohexylamino} -methyl}-1*H*-indol-2-carbonsäure,
4,6-Dichlor- 3- {[3'-(N, N-dimethylaminomethyl)- 4'-hydroxy- 4'-(3"-methoxyphenyl) -cyclohexylamino] -methyl}- 1 *H*-indol-2-carbonsäure,
1-*tert*-Butoxycarbonyl-4,6-dichlor-3-{[3'-(N,N-dimethylaminomethyl)-4'-hydroxy-4'-(3"-methoxyphenyl)-cyclohexylamino]-methyl}-indol-2-carbonsäureethylester,
4,6-Dichlor- 3- { [3'-(N.N-dimethylaminomethyl)- 4'-hydroxy- 4'-(3"-mehtoxyphenyl) -cyctohexylamino] -methyl}-1-methyl-indol-2-carbonsäureethylester,
4,6-Dichlor- 3- { [3'-(N.N-dimethylaminomethyl)- 4'-hydroxy- 4'-(3"-methoxyphenyl) -cyclohexylamino] -methyl}-1-benzyl-indol-2-carbonsäureethylester,
5-Chlor-3-{[3'-(N,N-dimethylaminomethyl)-4'-hydroxy-4'-(3"-methoxyphenyl)-cyclohexlyamino]-methyl}-1-benzyl-indol-2-carbonsäureethylester,

gegebenenfalls In Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säuren oder ihrer Basen oder in Form ihrer

Salze, insbesondere der physiologisch verträglichen Salze, oder in Form ihrer Solvate, insbesondere der Hydrate.

**14.** Verfahren zur Herstellung von substituierten Indolen gemäß einem der Ansprüche 1-13, bei dem man

A) gegebenenfalls ein Indol der Formel Y-$R^x$, worin $R^x$ für Wasserstoff oder eine Gruppe der Formeln $(CH_2)_nOH$ oder $(CH_2)_nNR^{1'}H$ steht, worin n für 0, 1, 2 oder 3 steht und $R^{1'}$ die Bedeutung gemäß Anspruch 1 hat, und Y für einen Rest der allgemeinen Formel Y steht, worin der freie Strich die Bindung zum Rest $R^x$ symbolisiert und worin $R^1$, $R^2$, $R^3$, $R^4$, $R^5$ und $R^6$ die vorstehend genannte Bedeutung haben, derivatisiert, **dadurch** daß man

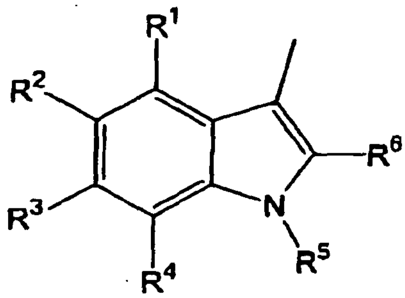

Y

a) ein Indol der Formel Y-H mit einem N,N-disubstituierten Formamid, vorzugsweise N-Methyl-N-phenylformamid, in Gegenwart von Phosphoroxidchlorid in einem geeigneten Lösungsmittel, vorzugsweise 1,2-Dichlorethan, zu dem entsprechenden Aldehyd der Formel Y-CHO umsetzt,
b) einen Aldehyd der Formel Y-CHO gemäß Schritt a) mit Hilfe von Reduktionsmitteln, vorzugsweise Natriumcyanoborhydrid oder $NaBH_2S_3$, in einem geeigneten Lösungsmittel, gegebenenfalls in Gegenwart eines Puffers und unter Kühlung zu dem entsprecheneden Alkohol der Formel Y-$CH_2$-OH umgesetzt,
c) einen Alkohol der Formel Y-$(CH_2)_n$-OH gemäß Schritt b) oder A) mit einem Bromierungsmittel, vorzugsweise $PBr_3$ oder $Ph_3PBr_2$, zu dem entsprechenden Bromid der Formel Y-$(CH_2)_n$-Br umsetzt,

und anschließend aufarbeitet und gegebenenfalls das Produkt reinigt,
C) gegebenenfalls eine Verbindung der Formel X-R', worin X die vorstehend genannte Bedeutung hat und R' für eine funktionelle Gruppe steht, derivatisiert **dadurch** daß man

a) ein Keton der Formel X=O 1) mit Methoxymethyltriphenylphosphiniumchlorid unter Schutzgas in einem geeigneten Lösungsmittel, vorzugsweise in Dimethylformamid, in Gegenwart von Natriumhydrid und anschließend mit Salzsäure oder 2) mit $Me_3S^+BF_4^-$ zu dem entsprechenden, um ein Kohlenstoffatom verlängerten Aldehyd X-CHO umsetzt,
b) einen Aldehyd der Formel X-CHO gemäß a) mit einem Reduktionsmittel, vorzugsweise Natriumborhydrid, in einem geeigneten Lösungsmittel, vorzugsweise einem Ethanol/Wasser-Gemisch zu dem entsprechenden Alkohol X-$CH_2$-OH umsetzt.
c) einen Alkohol X-$CH_2$-OH gemäß b) oder der Formel X-OH mit einem Bromienrugsmittel, vorzugsweise Triphenylphosphindibromid, in einem geeigneten Lösungsmittel, vorzugsweise Acetonitril, zu dem entsprechenden Bromid der Formel X-$CH_2$-Br bzw. X-Br umsetzt.
d) ein Bromid der Formel X-$CH_2$-Br gemäß c) mit einem Phosphin der Formel $PR''_3$, worin R" für einen organischen Rest steht, vorzugsweise für einen Phenylrest, in einem geeigneten Lösungsmittel, vorzugsweise Toluol, Ether, Tetrahydrofuran oder Aceton, unter Kühlung und Schutzgas zu dem entsprechenden Phosphonlumsalz $R''_3P^+$-$CHX^-$ umsetzt,
e) ein Bromid der Formel X-$CH_2$-Br gemäß c) mit einem Phosphit der Formel $HP(O)(OR''')_2$, worin R''' für einen organischen Rest steht, bei erhöhter Temperatur, vorzugsweise 200°C, zu dem entsprechenden Phosphonat $(R'''O)_2P(O)$-$CH_2$-X umsetzt

und anschließend aufarbeitet und gegebenenfalls das Produkt reinigt,
D) eine Verbindung der Formel Y-$R^x$ oder dessen Derivat aus dem Schritt A), worin Y die vorstehend genannte Bedeutung hat, mit einer Verbindung der Formel der Formel X-R' oder dessen Derivat aus dem Schritt C), worin X und R' die vorstehend genannte Bedeutung haben, umsetzt, **dadurch** daß man

f) ein Amin der Formel Y-$(CH_2)_n$-$NHR^{1'}$ mit einem Bromid der Formel X-Br in Gegenwart eines geeigneten Katalysators, vorzugsweise Caesiumcarbonat, in einem geeignten Lösungsmittel, vorzugsweise Dimethylformamid, unter Ausbildung einer Aminobrücke umsetzt,

g) ein Bromid der Formel Y-$(CH_2)_n$-Br mit einem Amin der Formel X- $NHR^{1'}$ in Gegenwart eines geeigneten Katalysators, vorzugsweise Caesiumcarbonat, in einem geeignten Lösungsmittel, vorzugsweise Dimethylformamid, unter Ausbildung einer Aminobrücke umsetzt,

h) einen Aldehyd der Formel Y-CHO mit einem Amin der Formel X-$NHR^{1'}$ in Gegenwart eines geeigneten Reduktionsmittels, vorzugsweise Natriumcyanoborhydrid und Natriumtriacetoxyborhydrid, in einem geeigneten Lösungsmittel, vorzugsweise einem Gemisch aus Tetrahydrofuran und 1,2-Dichlorethan, unter Ausbildung einer-$CH_2$-$NR^{1'}$-Brücke umsetzt,

i) einen Aldehyd der Formel Y-CHO mit einem Phosphoniumsalz $R''_3P^+$-$CHX^-$, worin R" die vorstehend genannte Bedeutung hat, unter Schutzgas in Gegenwart geeigneter Katalysatoren in einem geeigneten Lösungsmittel, vorzugsweise in Gegenwart von Natriummethanolat in einem Gemisch aus Hexan, Diethylether und/oder Diisopropylether oder in Gegenwart von Natriumhydrid, Kallum-tert-butylat oder einem Lithiumamid In Dimethylformamid oder Dimethylsulfoxid, unter Ausbildung einer -CH=CH-Brücke umsetzt oder

j) einen Aldehyd der Formel Y-CHO mit einem Phosphonat der Formel $(R'''O)_2P(O)$-$CH_2$-X, worin R"' die vorstehend genannte Bedeutung hat, unter Schutzgas in Gegenwart geeigneter Katalysatoren, vorzugsweise Natriummethanolat, Natriumhydroxid, Kaliumhydroxid. Natriumhydrid, Kalium-tert-butylat oder einem Lithiumamid, in einem geeigneten Lösungsmittel, vorzugsweise Dimethylformamid, Dimethylsufoxid, Diethylether, Tetrahydrofuran, unter Ausbildung einer -CH=CH-Brücke umsetzt,

k) gegebenenfalls die -CH=CH-Brücke aus dem Schritt i) oder j) durch Wasserstoff, vorzugsweise bei Normaldruck oder erhöhtem Druck bis zu 100 bar, in Gegenwart geeigneter Katalysatoren, vorzugsweise Übergangsmetallen oder Übergangsmetallverbindungen, vorzugsweise Palladium oder seine Salze, Rhodium oder seine Komplexe, in einem geeigneten Lösungsmittel, vorzugsweise Dimethylformamid, Methanol oder Ethanol, bei einer Temperatur zwischen 20 und 100°C unter Ausbildung einer -$CH_2$-$CH_2$Brücke hydriert, und anschließend aufarbeitet und gegebenenfalls das Produkt reinigt,

E) gegebenenfalls einen Indol-2-carbonsäureester der Formel Y-A-X, worin Y, A und X die vorstehend genannte Bedeutung haben, wobei $R^6$ in Y für eine Gruppe der Formel $COR^7$ steht, worin $R^7$ für die Gruppe $OR^8$ steht und $R^8$ die vorstehend genannte Bedeutung mit Ausnahme von Wasserstoff hat, in Gegenwart einer Base, vorzugsweise Kalium- oder Natnum-Hydroxid, in einem geeigneten Lösungsmittel, vorzugsweise einem Alkohol/Wassergemisch, besonders bevorzugt in einem Methanol oder Ethanol/Wassergemisch, verseift und anschließend aufarbeitet und gegebenenfalls die Indol-2-carbonsäune der Formel Y-A-X, worin $R^6$ in Y für eine Gruppe der Formel $COR^7$ steht, worin $R^7$ für die Gruppe $OR^8$ und $R^8$ für Wasserstoff steht, reinigt.

**15.** Arzneimittel enthaltend wenigstens eine substituierte Indolverbindung, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, gemäß einem der Ansprüche 1 bis 13 und gegebenenfalls physiologisch verträgliche Hilfsstoffe.

**16.** Arzneimittel gemäß Anspruch 15 zur Bekämpfung von Schmerz.

**17.** Arzneimittel gemäß Anspruch 16 zur Bekämpfung von chronischem Schmerz

**18.** Arzneimittel gemäß Anspruch 15 oder 16 zur Bekämpfung von neuropathischem Schmerz.

**19.** Arzneimittel gemäß Anspruch 15 zur Behandlung oder Prophylaxe von neurodegenerativen Erkrankungen, vorzugsweise von Morbus Alzheimer, Morbus Parkinson oder Morbus Huntington.

**20.** Arzneimittel gemäß Anspruch 15 zur Behandlung oder Prophylaxe von Schlaganfall.

**21.** Arzneimittel gemäß Anspruch 15 zur Behandlung oder Prophylaxe von cerebraler Ischämie.

**22.** Arzneimittel gemäß Anspruch 15 zur Behandlung oder Prophylaxe von cerebralem Infarkt.

**23.** Arzneimittel gemäß Anspruch 15 zur Behandlung oder Prophylaxe von Himödem.

**24.** Arzneimittel gemäß Anspruch 15 zur Behandlung oder Prophylaxe von Unterversorgungszuständen des zentralen Nervensystems, vorzugsweise Hypoxie oder Anoxie.

**25.** Arzneimittel gemäß Anspruch 15 zur Behandlung oder Prophylaxe von Epilepsie.

**26.** Arzneimittel gemäß Anspruch 15 zur Behandlung oder Prophylaxe von Schizophrenie.

**27.** Arzneimittel gemäß Anspruch 15 zur Behandlung oder Prophylaxe von Psychosen bedingt durch erhöhten Aminosäurespiegel.

**28.** Arzneimittel gemäß Anspruch 15 zur Behandlung oder Prophylaxe von AIDS-Demenz.

**29.** Arzneimittel gemäß Anspruch 15 zur Behandlung oder Prophylaxe des Tourette-Syndroms.

**30.** Arzneimittel gemäß Anspruch 15 zur Behandlung oder Prophylaxe von Encephalomyelitis.

**31.** Arzneimittel gemäß Anspruch 15 zur Behandlung oder Prophylaxe von perinataler Asphyxie.

**32.** Arzneimittel gemäß Anspruch 15 zur Behandlung oder Prophylaxe von Tinnitus.

**33.** Arzneimittel gemäß Anspruch 15 zur Behandlung oder Prophylaxe von Migräne.

**34.** Arzneimittel gemäß Anspruch 15 zur Behandlung oder Prophylaxe von inflammatorischen und/oder allergische Reaktionen.

**35.** Arzneimittel gemäß Anspruch 15 zur Behandlung oder Prophylaxe von Depressionen.

**36.** Arzneimittel gemäß Anspruch 15 zur Behandlung oder Prophylaxe von seelischen Erkrankungen.

**37.** Arzneimittel gemäß Anspruch 15 zur Behandlung oder Prophylaxe von Haminkontinenz.

**38.** Arzneimittel gemäß Anspruch 15 zur Behandlung oder Prophylaxe von Juckreiz.

**39.** Arzneimittel gemäß Anspruch 15 zur Behandlung oder Prophylaxe von Diarrhoe.

**40.** Arzneimittel gemäß Anspruch 15 zur Anxiolyse.

**41.** Arzneimittel gemäß Anspruch 15 zur Anästhesie.

**42.** Verwendung wenigstens eines substituierten Indols, gegebenenfalls in Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes,insbesondere eines physiologisch verträglichen Salzes, oder in Form ihres Solvates, insbesondere des Hydrates, gemäß einem der Ansprüche 1 bis 13 zur Herstellung eines Arzneimittels zur Bekämpfung von Schmerz, vorzugsweise chronischem oder neuropathischem Schmerz.

**43.** Verwendung wenigstens eines substituierten Indols, gegebenenfalls In Form ihres Racemates, ihres reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, oder jeweils in Form ihrer Säure oder ihrer Base oder in Form ihres Salzes, insbesondere eines physiologisch verträglichen Salzes, oder in Form ihres Solvates, insbesondere des Hydrates, gemäß einem der Ansprüche 1 bis 13 zur Herstellung eines Arzneimittels zur Behandlung oder Prophylaxe von neurodegenerativen Erkrankungen, vorzugsweise von Morbus Alzheimer, Morbus Parkinson oder Morbus Huntington, zur Behandlung oder Prophylaxe von Schlaganfall, cerebraler Ischämie, cerebralem Infarkt, Hlmödem, Unterversorgungszuständen des zentralen Nervensystems, vorzugsweise Hypoxie oder Anoxie, Epilepsie, Schizophrenie, Psychosen bedingt durch erhöhten Aminosäurespiegel, AIDS-Demenz, Encephalomyelitis, Tourette-Syndrom, perinataler Asphyxie, Tinnitus. Migräne, inflammatorischen und/oder allergischen Reaktionen, Depressionen, seelischen Erkrankungen, Haminkontinenz, Juckreiz oder Diarrhoe oder zur Anxiolyse oder Anästhesie.

## Claims

1. Substituted indoles of the general formula I,

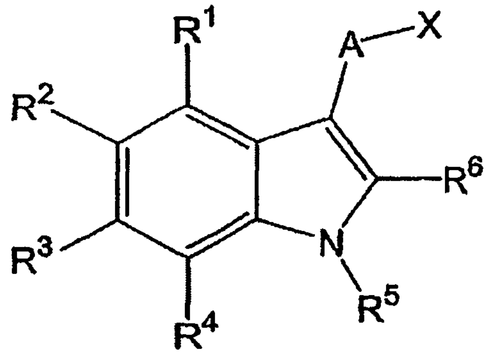

I

in which
$R^1$, $R^2$, $R^3$ and $R^4$, identical or different, denote a linear or branched, saturated or unsaturated aliphatic $C_{1-10}$ residue or a saturated or unsaturated cycloaliphatic $C_{3-7}$ residue, wherein each of the above-stated residues may optionally be joined via an ether bridge, or hydrogen, a halogen or a hydroxy group,
$R^5$ denotes hydrogen, a linear or branched, saturated or unsaturated aliphatic $C_{1-10}$ residue or a saturated or unsaturated cycloaliphatic $C_{3-7}$ residue, an aryl or heteroaryl residue, wherein the aryl or heteroaryl residue may be optionally joined via a $C_{1-6}$ alkylene group, a substituted sulfonyl residue or a group of the formula $-COR^7$, wherein $R^7$ has the meaning stated hereinafter,
$R^6$ denotes a group of the formula $-COR^7$ wherein $R^7$ has the meaning stated hereinafter,
$R^7$denotes the group $OR^8$, wherein the residue $R^8$ has the meaning stated hereinafter,
$R^8$ denotes hydrogen, or a linear or branched, saturated or unsaturated aliphatic $C_{1-10}$ residue,
A denotes a bridge with the formula $-(CH_2)_2-$ or $-(CH_2)NR^{1'}-$, in which n denotes 0, 1, 2 or 3, $R^{1'}$ has the meaning stated hereinafter and the bond to the residue X is always stated last,
and X denotes the following residue, in which the unoccupied bond line symbolises the bond to the bridge A

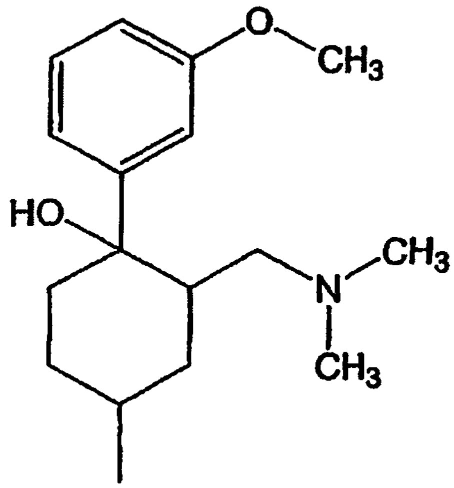

and
$R^{1'}$ denotes hydrogen or a linear or branched, saturated or unsaturated aliphatic $C_{1-10}$ residue,
optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

2. Substituted indoles according to claim 1, **characterised in that** $R^2$ denotes a linear or branched, saturated or unsaturated aliphatic $C_{1-3}$ residue or a halogen and $R^1$, $R^3$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

3. Substituted indoles according to claim 1, **characterised in that** $R^3$ denotes a linear or branched, saturated or unsaturated aliphatic $C_{1-3}$ residue or a halogen and $R^1$, $R^2$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

4. Substituted indoles according to claim 1, **characterised in that** $R^2$ and $R^3$, identical or different, denote a linear or branched, saturated or unsaturated aliphatic $C_{1-3}$ residue or a halogen and $R^1$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

5. Substituted indoles according to claim 1, **characterised in that** $R^1$ and $R^3$, identical or different, denote a linear or branched, saturated or unsaturated aliphatic $C_{1-3}$ residue or a halogen and $R^2$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

6. Substituted indoles according to claim 1, **characterised in that** $R^2$ denotes a methyl residue or a chlorine and $R^1$, $R^3$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

7. Substituted indoles according to claim 1, **characterised in that** $R^3$ denotes a methyl residue or a chlorine and $R^1$, $R^2$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

8. Substituted indoles according to claim 1, **characterised in that** $R^2$ and $R^3$ in each case denote a methyl residue or a chlorine and $R^1$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

9. Substituted indoles according to claim 1, **characterised in that** $R^1$ and $R^3$ in each case denote a methyl residue or a chlorine and $R^2$ and $R^4$ in each case denote hydrogen, optionally in the form of the racemates thereof, the pure stereoisomers thereof, in particular enantiomers or diastereomers, or in the form of mixtures of the stereoisomers, in particular the enantiomers or

diastereomers, in any desired mixing ratio or in each case in the form of the acids or bases thereof or in the form of the salts thereof, in particular physiologically acceptable salts, or in the form of the solvates thereof, in particular the hydrates.

**10.** Substituted indoles according to claim 1,
**characterised in that** $R^5$ denotes hydrogen, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

**11.** Substituted indoles according to claim 1,
**characterised in that** $R^6$ denotes a group of the formula $COR^7$, wherein $R^7$ denotes the group $OR^8$ and the residue $R^8$ denotes hydrogen or a linear or branched, saturated or unsaturated aliphatic $C_{1-3}$ residue, preferably a methyl or ethyl group, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

**12.** Substituted indoles according to claim 1,
**characterised in that** A denotes a bridge with one of the following formulae: $-CH_2-$, $-CH_2NR^{1'}-$, in which $R^{1'}$ denotes hydrogen or a linear or branched, saturated or unsaturated aliphatic $C_{1-3}$ group, optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

**13.** A substituted indole according to claim 1, selected from the group comprising:

5-Methyl- 3- { [3'-(N, N-dimethylaminomethyl)- 4'-hydroxy- 4'-(3''-methoxyphenyl) -cyclohexylamino] -methyl}-1H-indole 2-carboxylic acid methyl ester,
4,6-Dimethyl-3-{[3'-(N,N-dimethylaminomethyl)-4'-hydroxy-4'-(3''-methoxyphenyl)-cyclohexylamino]-methyl}-1H-indole 2-carboxylic acid ethyl ester,
5-Chloro- 3- { [3'-(N, N-dimethylaminomethyl)- 4'-hydroxy- 4'-(3''-methoxyphenyl) -cyclohexylamino] -methyl}-1H-indole 2-carboxylic acid ethyl ester,
4,6-Dichloro-3-{[3'-(N,N-dimethylaminomethyl)-4'-hydroxy-4'-(3''-methoxyphenyl)-cyclohexylamino]-methyl}-1H-indole 2-carboxylic acid ethyl ester,
4,6-Dimethyl-3-{[3'-(N,N-dimethylaminomethyl)-4'-hydroxy-4'-(3''-methoxyphenyl)-cyclohexyl-(N-methylamino)]-methyl}-1H-indole 2-carboxylic acid ethyl ester
4,6-Dichloro-3-{[3'-(N,N-dimethylaminomethyl)-4'-hydroxy-4'-{3''-methoxyphenyl)-cyclohexyl-(N-methylamino)]-methyl}-1H-indole 2-carboxylic acid ethyl ester,
5-Chloro-3-{[3'-(N, N-dimethylaminomethyl)- 4'-hydroxy- 4'-(3''-methoxyphenyl) -cyclohexyl-(N-methylamino)] -methyl}-1H-indole 2-carboxylic acid ethyl ester,
5-Methyl- 3- { [3'-(N, N-dimethylaminomethyl)- 4'-hydroxy- 4'-(3''-methoxyphenyl) -cyclohexylamino] -methyl}-1H-indole 2-carboxylic acid,
5-Chloro- 3- { [3'-(N, N-dimethylaminomethyl)- 4'-hydroxy- 9'-(3''-methoxyphenyl) -cyclohexylamino] -methyl}-1H-indole 2-carboxylic acid,
4,6-Dimethyl-3-{[3'-(N,N-dimethylaminomethyl)-4'-hydroxy-4'-(3''-methoxyphenyl)-cyclohexylamino]-methyl}-1H-indole 2-carboxylic acid,
4,6-Dichloro-3-{[3'-(N,N-dimethylaminomethyl)-4'-hydroxy-4'-(3''-methoxyphenyl)-cyclohexylamino]-methyl}-1H-indole 2-carboxylic acid,
1-tert-Butoxycarbonyl- 4,6-dichloro- 3- { [3'-N, N-dimethylaminomethyl)- 4'-hydroxy- 4'-(3''-methoxyphenyl) -cyclohexylamino]-methyl}-indole 2-carboxylic acid ethyl ester,
4,6-Dichloro-3-{[3'-(N,N-dimethylaminomethyl)-4'-hydroxy-4'-(3''-methoxyphenyl)-cyclohexylamino]-methyl}-1-methyl-indole 2-carboxylic acid ethyl ester,
4,6-Dichloro-3-{[3'-(N,N-dimethylaminomethyl)-4'-hydroxy-4'-(3''-methoxyphenyl)-cyclohexylamino]-methyl}-1-benzyl-indole 2-carboxylic acid ethyl ester,
5-Chloro- 3- { [3'-(N, N-dimethylaminomethyl)- 4'-hydroxy- 4'-(3''-methoxyphenyl) -cyclohexylamino] -methyl}-1-benzyl-indole 2-carboxylic acid ethyl ester,

optionally in the form of the racemates thereof, the pure stereoisomers thereof, in particular enantiomers or diastereomers, or in the form of mixtures of the stereoisomers, in particular the enantiomers or diastereomers, in any desired mixing ratio or in each case in the form of the acids or bases thereof or in the form of the salts thereof, in particular physiologically acceptable salts, or in the form of the solvates thereof, in particular the hydrates.

**14.** A process for the production of substituted indoles according to any one of claims 1-13, in which

A) an indole of the formula Y-$R^x$ is optionally derivatised, in which $R^x$ denotes hydrogen or a group of the formulae $(CH_2)_nOH$ or $(CH_2)_nNR^{1'}H$, in which n denotes 0, 1, 2 or 3 and $R^{1'}$ has the meaning according to claim 1 and Y denotes a residue of the general formula Y, in which the unoccupied bond line symbolises the bond to the residue $R^x$ and in which $R^1$, $R^2$, $R^3$, $R^4$, $R^5$ and $R^6$ have the above-stated meaning, in that

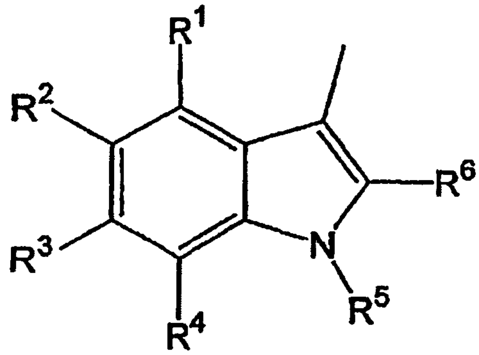

Y

a) an indole of the formula Y-H is reacted with an N,N-disubstituted formamide, preferably N-methyl-N-phenylformamide, in the presence of phosphorus oxychloride in a suitable solvent, preferably 1,2-dichloroethane, to yield the corresponding aldehyde of the formula Y-CHO,
b) an aldehyde of the formula Y-CHO according to step a) is reacted with the assistance of reducing agents, preferably sodium cyanoborohydride or $NaBH_2S_3$, in a suitable solvent, optionally in the presence of a buffer and with cooling to yield the corresponding alcohol of the formula Y-$CH_2$-OH,
c) an alcohol of the formula Y-$(CH_2)_n$-OH according to step b) or A) is reacted with a brominating agent, preferably $PBr_3$ or $Ph_3PBr_2$ to yield the corresponding bromide of the formula Y-$(CH_2)_n$-Br,

and is then worked up and the product is optionally purified,
C) a compound of the formula X-R', in which X has the above-stated meaning and R' denotes a functional group, is optionally derivatised in that

a) a ketone of the formula X=O is reacted 1) with methoxymethyl triphenylphosphinium chloride under protective gas in a suitable solvent, preferably in dimethylformamide, in the presence of sodium hydride and then with hydrochloric acid or 2) with $Me_3S^+BF_4^-$ to yield the corresponding aldehyde X-CHO extended by one carbon atom,
b) an aldehyde of the formula X-CHO according to a) is reacted with a reducing agent, preferably sodium borohydride, in a suitable solvent, preferably an ethanol/water mixture, to yield the corresponding alcohol X-$CH_2$-OH,
c) an alcohol X-$CH_2$-OH according to b) or of the formula X-OH is reacted with a brominating agent, preferably triphenylphosphine dibromide, in a suitable solvent, preferably acetonitrile, to yield the corresponding bromide of the formula X-$CH_2$-Br or X-Br,
d) a bromide of the formula X-$CH_2$-Br according to c) is reacted with a phosphine of the formula $PR''_3$, in which R" denotes an organic residue, preferably a phenyl residue, in a suitable solvent, preferably toluene, ether, tetrahydrofuran or acetone, with cooling and under protective gas to yield the corresponding phosphonium salt $R''_3P^+$-$CHX^-$,
e) a bromide of the formula X-$CH_2$-Br according to c) is reacted with a phosphite of the formula HP(O)$(OR''')_2$, in which R''' denotes an organic residue, at elevated temperature, preferably 200°C, to yield the corresponding phosphonate $(R'''O)_2P(O)$-$CH_2$-X

and is then worked up and the product is optionally purified,
D) a compound of the formula Y-$R^x$ or the derivative thereof from step A), in which Y has the above-stated meaning, is reacted with a compound of the formula X-R' or the derivative thereof from step C), in which X and R' have the above-stated meaning, in that

f) an amine of the formula Y- $(CH_2)_n$-$NHR^{1'}$ is reacted with a bromide of the formula X-Br in the presence of a suitable catalyst, preferably caesium carbonate, in a suitable solvent, preferably dimethylformamide, with formation of an amino bridge,
g) a bromide of the formula Y-$(CH_2)_n$-Br is reacted with an amine of the formula X-$NHR^{1'}$ in the presence of a suitable catalyst, preferably caesium carbonate, in a suitable solvent, preferably dimethylformamide, with formation of an amino bridge,
h) an aldehyde of the formula Y-CHO is reacted with an amine of the formula X-$NHR^{1'}$ in the presence of a suitable reducing agent, preferably sodium cyanoborohydride and sodium triacetoxyborohydride, in a suitable solvent, preferably a mixture of tetrahydrofuran and 1,2-dichloroethane, with formation of a -$CH_2$-$NR^{1'}$ bridge,
i) an aldehyde of the formula Y-CHO is reacted with a phosphonium salt $R''_3P^+$-$CHX^-$, in which R" has the above-stated meaning, under protective gas in the presence of suitable catalysts in a suitable solvent, preferably in the presence of sodium methanolate in a mixture of hexane, diethyl ether and/or diisopropyl ether or in the presence of sodium hydride, potassium tert-butylate or a lithium amide in dimethylformamide or dimethyl sulfoxide, with formation of a -CH=CH- bridge or
j) an aldehyde of the formula Y-CHO is reacted with a phosphonate of the formula $(R'''O)_2P(O)$-$CH_2$-X, in which R"' has the above-stated meaning, under protective gas in the presence of suitable catalysts, preferably sodium methanolate, sodium hydroxide, potassium hydroxide, sodium hydride, potassium tert-butylate or a lithium amide, in a suitable solvent, preferably dimethylformamide, dimethyl sulfoxide, diethyl ether, tetrahydrofuran, with formation of a -CH=CH- bridge,
k) the -CH=CH- bridge from step i) or j) is optionally hydrogenated by hydrogen, preferably at standard pressure or elevated pressure of up to 100 bar, in the presence of suitable catalysts, preferably transition metals or transition metal compounds, preferably palladium or the salts thereof, rhodium or the complexes thereof, in a suitable solvent, preferably dimethylformamide, methanol or ethanol, at a temperature of between 20 and 100°C with formation of a -$CH_2$-$CH_2$- bridge,

and is then worked up and the product is optionally purified,
E) an indole 2-carboxylic acid ester of the formula Y-A-X, in which Y, A and X have the above-stated meaning, wherein $R^6$ in Y denotes a group of the formula $COR^7$, in which $R^7$ denotes the group $OR^8$ and $R^8$ has the above-stated meaning with the exception of hydrogen, is optionally saponified in the presence of a base, preferably potassium or sodium hydroxide, in a suitable solvent, preferably an alcohol/water mixture, particularly preferably in a methanol/ or ethanol/water mixture and then worked up, followed optionally by purification of the indole 2-carboxylic acid of the formula Y-A-X, in which $R^6$ in Y denotes a group of the formula $COR^7$, in which $R^7$ denotes the group $OR^8$ and $R^8$ denotes hydrogen.

**15.** A pharmaceutical preparation containing at least one substituted indole compound, optionally in the form of the racemate thereof, the pure stereoisomer thereof, in particular enantiomers or diastereomers, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in the form of the solvate thereof, in particular the hydrate, according to any one of claims 1 to 13, and optionally physiologically acceptable auxiliary substances.

**16.** A pharmaceutical preparation according to claim 15 for combatting pain.

**17.** A pharmaceutical preparation according to claim 16 for combatting chronic pain.

**18.** A pharmaceutical preparation according to claim 15 or claim 16 for combatting neuropathic pain.

**19.** A pharmaceutical preparation according to claim 15 for the treatment or prevention of neurodegenerative diseases, preferably of Alzheimer's disease, Parkinson's disease or Huntington's chorea.

**20.** A pharmaceutical preparation according to claim 15 for the treatment or prevention of stroke.

**21.** A pharmaceutical preparation according to claim 15 for the treatment or prevention of cerebral ischaemia.

**22.** A pharmaceutical preparation according to claim 15 for the treatment or prevention of cerebral infarct.

**23.** A pharmaceutical preparation according to claim 15 for the treatment or prevention of cerebral oedema.

**24.** A pharmaceutical preparation according to claim 15 for the treatment or prevention of insufficiency states of the central nervous system, preferably hypoxia or anoxia.

**25.** A pharmaceutical preparation according to claim 15 for the treatment or prevention of epilepsy.

**26.** A pharmaceutical preparation according to claim 15 for the treatment or prevention of schizophrenia.

**27.** A pharmaceutical preparation according to claim 15 for the treatment or prevention of psychoses brought about by elevated amino acid levels.

**28.** A pharmaceutical preparation according to claim 15 for the treatment or prevention of AIDS dementia.

**29.** A pharmaceutical preparation according to claim 15 for the treatment or prevention of Tourette's syndrome.

**30.** A pharmaceutical preparation according to claim 15 for the treatment or prevention of encephalomyelitis.

**31.** A pharmaceutical preparation according to claim 15 for the treatment or prevention of perinatal asphyxia.

**32.** A pharmaceutical preparation according to claim 15 for the treatment or prevention of tinnitus.

**33.** A pharmaceutical preparation according to claim 15 for the treatment or prevention of migraine.

**34.** A pharmaceutical preparation according to claim 15 for the treatment or prevention of inflammatory and/or allergic reactions.

**35.** A pharmaceutical preparation according to claim 15 for the treatment or prevention of depression.

**36.** A pharmaceutical preparation according to claim 15 for the treatment or prevention of mental health conditions.

**37.** A pharmaceutical preparation according to claim 15 for the treatment or prevention of urinary incontinence.

**38.** A pharmaceutical preparation according to claim 15 for the treatment or prevention of pruritus.

**39.** A pharmaceutical preparation according to claim 15 for the treatment or prevention of diarrhoea.

**40.** A pharmaceutical preparation according to claim 15 for anxiolysis.

**41.** A pharmaceutical preparation according to claim 15 for anaesthesia.

**42.** Use of at least one substituted indole, optionally in the form of the racemate thereof, the pure stereoisomer thereof, in particular enantiomers or diastereomers, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in the form of the solvate thereof, in particular the hydrate, according to any one of claims 1 to 13 for the production of a pharmaceutical preparation for combatting pain, preferably of chronic or neuropathic pain.

**43.** Use of at least one substituted indole, optionally in the form of the racemate thereof, the pure stereoisomer thereof, in particular enantiomers or diastereomers, or in the form of mixtures of the stereoisomers, in particular the enantiomers or diastereomers, in any desired mixing ratio or in each case in the form of the acid or base thereof or in the form of the salt thereof, in particular a physiologically acceptable salt, or in the form of the solvate thereof, in particular the hydrate, according to any one of claims 1 to 13 for the production of a pharmaceutical preparation for the treatment or prevention of neurodegenerative diseases, preferably Alzheimer's disease, Parkinson's disease

or Huntington's chorea, for the treatment or prevention of stroke, cerebral ischaemia, cerebral infarct, cerebral oedema, insufficiency states of the central nervous system, preferably hypoxia or anoxia, epilepsy, schizophrenia, psychoses brought about by elevated amino acid levels, AIDS dementia, encephalomyelitis, Tourette's syndrome, perinatal asphyxia, tinnitus, migraine, inflammatory and/or allergic reactions, depression, mental health conditions, urinary incontinence, pruritus or diarrhoea or for anxiolysis or anaesthesia.

## Revendications

**1.** Indoles substitués de formule générale I,

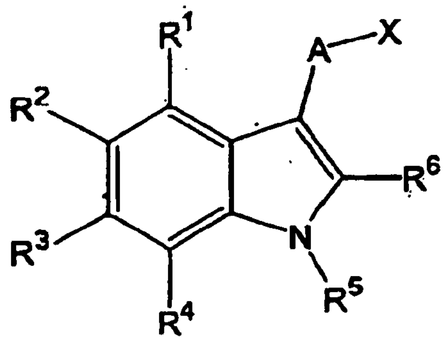

I

dans laquelle
$R^1$, $R^2$, $R^3$ et $R^4$, sont identiques ou différents et représentent un radical aliphatique en $C_1$ à $C_{10}$ linéaire ou ramifié, saturé ou insaturé ou un radical cycloaliphatique en $C_3$ à $C_7$ saturé ou insaturé, chacun des radicaux susmentionnés pouvant le cas échéant être lié via un pont éther, ou hydrogène, un halogène ou un groupe hydroxy,
$R^5$ représente hydrogène, un radical aliphatique en $C_1$ à $C_{10}$ linéaire ou ramifié, saturé ou insaturé ou un radical cycloaliphatique en $C_3$ à $C_7$ saturé ou insaturé, un radical aryle ou hétéroaryle, le radical aryle ou hétéroaryle pouvant le cas échéant être lié via un groupe alkylène en $C_1$ à $C_6$, un radical sulfonyle substitué ou un groupe de formule $-COR^7$, $R^7$ ayant la signification indiquée ci-dessous,
$R^6$ représente un groupe de formule $-COR^7$, $R^7$ ayant la signification indiquée ci-dessous,
$R^7$ représente le groupe $OR^8$, le radical $R^8$ ayant la signification indiquée ci-dessous,
$R^8$ représente hydrogène ou un radical aliphatique en $C_1$ à $C_{10}$ linéaire ou ramifié, saturé ou insaturé,
A représente un pont de formule $-(CH_2)_2-$ ou $-(CH_2)_nNR^{1'}-$, dans laquelle n représente 0, 1, 2 ou 3, $R^{1'}$ a la signification indiquée ci-dessous et la liaison au radical X est toujours mentionnée en dernier lieu,
et X représente le radical suivant, le trait libre symbolisant la liaison au pont A

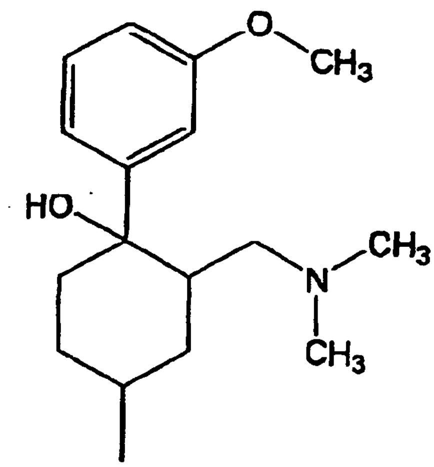

et
$R^{1'}$ représente hydrogène ou un radical aliphatique en $C_1$ à $C_{10}$ linéaire ou ramifié, saturé ou insaturé,

le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**2.** Indoles substitués selon la revendication 1, **caractérisés en ce que** $R^2$ représente un radical aliphatique en $C_1$ à $C_3$ linéaire ou ramifié, saturé ou insaturé ou un halogène et $R^1$, $R^3$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**3.** Indoles substitués selon la revendication 1, **caractérisés en ce que** $R^3$ représente un radical aliphatique en $C_1$ à $C_3$ linéaire ou ramifié, saturé ou insaturé ou un halogène et $R^1$, $R^2$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**4.** Indoles substitués selon la revendication 1, **caractérisés en ce que** $R^2$ et $R^3$ sont identiques ou différents et représentent un radical aliphatique en $C_1$ à $C_3$ linéaire ou ramifié, saturé ou insaturé ou un halogène et $R^1$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**5.** Indoles substitués selon la revendication 1, **caractérisés en ce que** $R^1$ et $R^3$ sont identiques ou différents et représentent un radical aliphatique en $C_1$ à $C_3$ linéaire ou ramifié, saturé ou insaturé ou un halogène et $R^2$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**6.** Indoles substitués selon la revendication 1, **caractérisés en ce que** $R^2$ représente un radical méthyle ou un chlore et $R^1$, $R^3$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**7.** Indoles substitués selon la revendication 1, **caractérisés en ce que** $R^3$ représente un radical méthyle ou un chlore et $R^1$, $R^2$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**8.** Indoles substitués selon la revendication 1, **caractérisés en ce que** $R^2$ et $R^3$ représentent à chaque fois un radical méthyle ou un chlore et $R^1$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**9.** Indoles substitués selon la revendication 1, **caractérisés en ce que** $R^1$ et $R^3$ représentent à chaque fois un radical méthyle ou un chlore et $R^2$ et $R^4$ représentent à chaque fois hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**10.** Indoles substitués selon la revendication 1, **caractérisés en ce que** $R^5$ représente hydrogène, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**11.** Indoles substitués selon la revendication 1, **caractérisés en ce que** $R^6$ représente un groupe de formule $COR^7$, $R^7$ représentant le groupe $OR^8$ et le radical $R^8$ représentant hydrogène ou un radical aliphatique en $C_1$ à $C_3$ linéaire ou ramifié, saturé ou insaturé, de préférence un groupe méthyle ou éthyle, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**12.** Indoles substitués selon la revendication 1, **caractérisés en ce que** A représente un pont présentant une des formules suivantes : $-CH_2-$, $-CH_2NR^{1'}-$, dans laquelle $R^{1'}$ représente hydrogène ou un groupe aliphatique en $C_1$ à $C_3$ linéaire ou ramifié, saturé ou insaturé, le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**13.** Indole substitué selon la revendication 1, choisi dans le groupe comprenant :

l'ester méthylique de l'acide 5-méthyl-3-{[3'-(N,N-diméthylaminométhyl)-4'-hydroxy-4'-(3"-méthoxyphényl)-cyclohexylamino]-méthyl}-1H-indole-2-carboxylique,
l'ester éthylique de l'acide 4,6-diméthyl-3-{[3'-(N,N-diméthylaminométhyl)-4'-hydroxy-4'-(3"-méthoxyphényl)-cyclohexylamino]-méthyl}-1H-indole-2-carboxylique,
l'ester éthylique de l'acide 5-chloro-3-{[3'-(N,N-diméthylaminométhyl)-4'-hydroxy-4'-(3"-méthoxyphényl)-cyclohexylamino]-méthyl}-1H-indole-2-carboxylique,
l'ester éthylique de l'acide 4,6-dichloro-3-{[3'-(N,N-diméthylaminométhyl)-4'-hydroxy-4'-(3"-méthoxyphényl)-cyclohexylamino]-méthyl}-1H-indole-2-carboxylique,
l'ester éthylique de l'acide 4,6-diméthyl-3-{[3'-(N,N-diméthylaminométhyl)-4'-hydroxy-4'-(3"-méthoxyphényl)-cyclohexyl-(N-méthylamino)]-méthyl}-1H-indole-2-carboxylique,
l'ester éthylique de l'acide 4,6-dichloro-3-{[3'-(N,N-diméthylaminométhyl)-4'-hydroxy-4'-(3"-méthoxyphényl)-cyclohexyl-(N-méthylamino)]-méthyl}-1H-indole-2-carboxylique,
l'ester éthylique de l'acide 5-chloro-3-{[3'-(N,N-diméthylaminométhyl)-4'-hydroxy-4'-(3"-méthoxyphényl)-cyclohexyl-(N-méthylamino)]-méthyl}-1H-indole-2-carboxylique,
l'acide 5-méthyl-3-{[3'-(N,N-diméthylaminométhyl)-4'-hydroxy-4'-(3"-méthoxyphényl)-cyclohexylamino]-méthyl}-1H-indole-2-carboxylique,
l'acide 5-chloro-3-{[3'-(N,N-diméthylaminométhyl)-4'-hydroxy-4'-(3"-méthoxyphényl)-cyclohexylamino]-méthyl}-1H-indole-2-carboxylique,
l'acide 4,6-diméthyl-3-{[3'-(N,N-diméthylaminométhyl)-4'-hydroxy-4'-(3"-méthoxyphényl)-cyclohexylamino]-méthyl}-1H-indole-2-carboxylique,
l'acide 4,6-dichloro-3-{[3'-(N,N-diméthylaminométhyl)-4'-hydroxy-4'-(3"-méthoxyphényl)-cyclohexylamino]-méthyl}-1H-indole-2-carboxylique,
l'ester éthylique de l'acide 1-tert-butoxycarbonyl-4,6-dichloro-3-{[3'-(N,N-diméthylaminométhyl)-4'-hydroxy-4'-(3"-méthoxyphényl)-cyclohexylamino]-méthyl}-indole-2-carboxylique,
l'ester éthylique de l'acide 4,6-dichloro-3-{[3'-(N,N-diméthylaminométhyl)-4'-hydroxy-4'-(3"-méthoxyphényl)-cyclohexylamino]-méthyl}-1-méthyl-indole-2-carboxylique,

l'ester éthylique de l'acide 4,6-dichloro-3-{[3'-(N,N-diméthylaminométhyl)-4'-hydroxy-4'-(3''-méthoxyphényl)-cyclohexylaminol]-méthyl}-1-benzyl-indole-2-carboxylique,
l'ester éthylique de l'acide 5-chloro-3-{[3'-(N,N-diméthylaminométhyl)-4'-hydroxy-4'-(3''-méthoxyphényl)-cyclohexylamino]-méthyl}-1-benzyl-indole-2-carboxylique,

le cas échéant sous forme de leurs racémates, leurs stéréo-isomères purs, en particulier leurs énantiomères ou diastéréo-isomères, ou sous forme de mélanges des stéréo-isomères, en particulier des énantiomères ou des diastéréo-isomères, dans un rapport de mélange quelconque ou à chaque fois sous forme de leurs acides ou de leurs bases ou sous forme de leurs sels, en particulier des sels physiologiquement acceptables, ou sous forme de leurs solvates, en particulier des hydrates.

**14.** Procédé pour la préparation d'indoles substitués selon l'une quelconque des revendications 1 à 13, dans lequel

A) on dérive le cas échéant un indole de formule Y-$R^x$, dans laquelle $R^x$ représente hydrogène ou un groupe de formules $(CH_2)_nOH$ ou $(CH_2)_nNR^{1'}H$, dans lesquelles n représente 0, 1, 2 ou 3 et $R^{1'}$ a la signification selon la revendication 1, et Y représente un radical de formule générale Y, dans laquelle le trait libre symbolise la liaison au radical $R^x$ et dans laquelle $R^1$, $R^2$, $R^3$, $R^4$, $R^5$ et $R^6$ ont la signification susmentionnée,

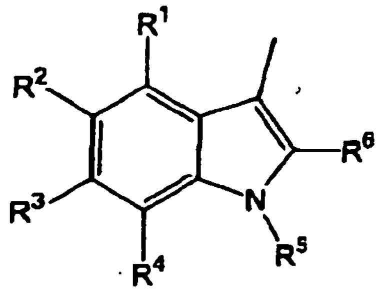

Y

a) en ce qu'on transforme un indole de formule Y-H avec un formamide N,N-disubstitué, de préférence le N-méthyl-N-phénylformamide, en présence de chlorure d'oxyde de phosphine dans un solvant approprié, de préférence le 1,2-dichloroéthane, en aldéhyde correspondant de formule Y-CHO,
b) on transforme un aldéhyde de formule Y-CHO selon l'étape a) à l'aide de réducteurs, de préférence le cyanoborohydrure de sodium ou $NaBH_2S_3$, dans un solvant approprié, le cas échéant en présence d'un tampon et sous refroidissement, en alcool correspondant de formule Y-$CH_2$-OH,
c) on transforme un alcool de formule Y-$(CH_2)_n$-OH selon l'étape b) ou A) avec un agent de bromuration, de préférence le $PBr_3$ ou le $Ph_3PBr_2$, en bromure correspondant de formule Y-$(CH_2)_n$-Br,

puis on traite et on purifie le cas échéant le produit,
C) on dérive le cas échéant un composé de formule X-R', dans laquelle X a la signification susmentionnée et R' représente un groupe fonctionnel,

a) en ce qu'on transforme une cétone de formule X=O 1) avec du chlorure de méthoxyméthyltriphénylphosphinium sous gaz protecteur dans un solvant approprié, de préférence dans du diméthylformamide, en présence d'hydrure de sodium et ensuite avec de l'acide chlorhydrique ou 2) avec du $Me_3S^+BF_4^-$ en aldéhyde X-CHO correspondant, allongé d'un atome de carbone,
b) on transforme un aldéhyde de formule X-CHO selon a) avec un réducteur, de préférence le borohydrure de sodium, dans un solvant approprié, de préférence un mélange éthanol/eau en alcool correspondant X-$CH_2$-OH,
c) on transforme un alcool X-$CH_2$-OH selon b) ou de formule X-OH avec un agent de bromuration, de préférence le dibromure de triphénylphosphine, dans un solvant approprié, de préférence l'acétonitrile, en bromure correspondant de formule X-$CH_2$-Br ou X-Br,
d) on transforme un bromure de formule X-$CH_2$-Br selon c) avec une phosphine de formule $PR''_3$, dans laquelle R'' représente un radical organique, de préférence un radical phényle, dans un solvant approprié, de préférence le toluène, l'éther, le tétrahydrofuranne ou l'acétone, sous refroidissement et un gaz protecteur en sel de phosphonium correspondant $R''_3P^+$-$CHX^-$,
e) on transforme un bromure de formule X-$CH_2$-Br selon c) avec un phosphite de formule $HP(O)(OR''')_2$,

dans laquelle R"' représente un radical organique, à température élevée, de préférence 200°C, en phosphonate correspondant $(RO''')_2P(O)-CH_2-X$

puis on traite et on purifie le cas échéant le produit,
D) on transforme un composé de formule $Y-R^x$ ou son dérivé de l'étape A), dans laquelle Y a la signification susmentionnée, avec un composé de formule X-R' ou son dérivé de l'étape C), dans laquelle X et R' ont la signification susmentionnée,

f) en ce qu'on transforme une amine de formule $Y-(CH_2)_n-NHR^{1'}$ avec un bromure de formule X-Br en présence d'un catalyseur approprié, de préférence le carbonate de césium, dans un solvant approprié, de préférence le diméthylformamide, avec formation d'un pont amino,
g) on transforme un bromure de formule $Y-(CH_2)_n-Br$ avec une amine de formule $X-NHR^{1'}$ en présence: d'un catalyseur approprié, de préférence le carbonate de césium, dans un solvant approprié, de préférence le diméthylformamide, avec formation d'un pont amino,
h) on transforme un aldéhyde de formule Y-CHO avec une amine de formule $X-NHR^{1'}$ en présence d'un réducteur approprié, de préférence le cyanoborohydrure de sodium et le triacétoxyborohydrure de sodium, dans un solvant approprié, de préférence un mélange de tétrahydrofuranne et de 1,2-dichloroéthane, avec formation d'un pont $-CH_2-NR^{1'}$,
i) on transforme un aldéhyde de formule Y-CHO avec un sel de phosphonium $R''_3P^+-CHX^-$, où R" a la signification susmentionnée, sous gaz protecteur en présence de catalyseurs appropriés dans un solvant approprié, de préférence en présence de méthanolate de sodium dans un mélange d'hexane, de diéthyléther et/ou de diisopropyléther ou en présence d'hydrure de sodium, de tert-butylate de potassium ou d'un amide de lithium dans du diméthylformamide ou du diméthylsulfoxyde, avec formation d'un pont - CH=CH- ou
j) on transforme un aldéhyde de formule Y-CHO avec un phosphonate de formule $(R'''O)_2P(O)-CH_2-X$, dans laquelle R"' a la signification susmentionnée, sous gaz protecteur en présence de catalyseurs appropriés, de préférence le méthanolate de sodium, l'hydroxyde de sodium, l'hydroxyde de potassium, l'hydrure de sodium, le tert-butylate de potassium ou un amide de lithium, dans un solvant approprié, de préférence le diméthylformamide, le diméthylsulfoxyde, le diéthyléther, le tétrahydrofuranne, avec formation d'un pont -CH=CH-,
k) on hydrogène le cas échéant le pont -CH=CH- de l'étape i) ou j) avec de l'hydrogène, de préférence à pression normale ou une pression élevée jusqu'à 100 bars, en présence de catalyseurs appropriés, de préférence les métaux de transition ou les composés de métaux de transition, de préférence le palladium ou ses sels, le rhodium ou ses complexes, dans un solvant approprié, de préférence le diméthylformamide, le méthanol ou l'éthanol, à une température entre 20 et 100°C avec formation d'un pont $-CH_2-CH_2-$

puis on traite et on purifie le cas échéant le produit,
E) on saponifie le cas échéant un ester d'acide indole-2-carboxylique de formule Y-A-X, dans laquelle Y, A et X ont la signification susmentionnée, $R^6$ dans Y représentant un groupe de formule $COR^7$, dans laquelle $R^7$ représente le groupe $OR^8$ et $R^8$ a la signification susmentionnée, à l'exception d'hydrogène, en présence d'une base, de préférence l'hydroxyde de potassium ou de sodium, dans un solvant approprié, de préférence un mélange alcool/eau, de manière particulièrement préférée dans un mélange méthanol/eau ou éthanol/eau puis on traite et on purifie le cas échéant l'acide indole-2-carboxylique de formule Y-A-X, dans laquelle $R^6$ dans Y représente un groupe de formule $COR^7$, dans laquelle $R^7$ représente le groupe $OR^8$ et $R^8$ représente hydrogène.

**15.** Médicament contenant au moins un composé substitué de l'indole, le cas échéant sous forme de son racémate, ses stéréo-isomères purs, en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 13 et le cas échéant des adjuvants physiologiquement acceptables.

**16.** Médicament selon la revendication 15 destiné à lutter contre la douleur.

**17.** Médicament selon la revendication 16 destiné à lutter contre la douleur chronique.

**18.** Médicament selon la revendication 15 ou 16 destiné à lutter contre la douleur neuropathique.

**19.** Médicament selon la revendication 15 destiné au traitement ou à la prophylaxie de maladies de neuro-dégénérescence, de préférence la maladie d'Alzheimer, de Parkinson ou de Huntington.

**20.** Médicament selon la revendication 15 destiné au traitement ou à la prophylaxie d'une attaque.

**21.** Médicament selon la revendication 15 destiné au traitement ou à la prophylaxie d'une ischémie cérébrale.

**22.** Médicament selon la revendication 15 destiné au traitement ou à la prophylaxie d'un infarctus cérébral.

**23.** Médicament selon la revendication 15 destiné au traitement ou à la prophylaxie d'un oedème cérébral.

**24.** Médicament selon la revendication 15 destiné au traitement ou à la prophylaxie d'états de sous-approvisionnement du système nerveux central, de préférence de l'hypoxie ou de l'anoxie.

**25.** Médicament selon la revendication 15 destiné au traitement ou à la prophylaxie de l'épilepsie.

**26.** Médicament selon la revendication 15 destiné au traitement ou à la prophylaxie de la schizophrénie.

**27.** Médicament selon la revendication 15 destiné au traitement ou à la prophylaxie de psychoses provoquées par un niveau élevé d'acides aminés.

**28.** Médicament selon la revendication 15 destiné au traitement ou à la prophylaxie de la démence liée au SIDA.

**29.** Médicament selon la revendication 15 destiné au traitement ou à la prophylaxie du syndrome de Tourette.

**30.** Médicament selon la revendication 15 destiné au traitement ou à la prophylaxie de l'encéphalomyélite.

**31.** Médicament selon la revendication 15 destiné au traitement ou à la prophylaxie de l'asphyxie périnatale.

**32.** Médicament selon la revendication 15 destiné au traitement ou à la prophylaxie de Tinnitus.

**33.** Médicament selon la revendication 15 destiné au traitement ou à la prophylaxie de la migraine.

**34.** Médicament selon la revendication 15 destiné au traitement ou à la prophylaxie de réactions inflammatoires et/ou allergiques.

**35.** Médicament selon la revendication 15 destiné au traitement ou à la prophylaxie de dépressions.

**36.** Médicament selon la revendication 15 destiné au traitement ou à la prophylaxie de maladies psychiques.

**37.** Médicament selon la revendication 15 destiné au traitement ou à la prophylaxie de l'incontinence urinaire.

**38.** Médicament selon la revendication 15 destiné au traitement ou à la prophylaxie du prurit.

**39.** Médicament selon la revendication 15 destiné au traitement ou à la prophylaxie de la diarrhée.

**40.** Médicament selon la revendication 15 destiné à l'anxiolyse.

**41.** Médicament selon la revendication 15 destiné à l'anesthésie.

**42.** Utilisation d'au moins un indole substitué, le cas échéant sous forme de son racémate, ses stéréo-isomères purs, en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 13 pour la préparation d'un médicament destiné à lutter contre la douleur, de préférence la douleur chronique ou neuropathique.

**43.** Utilisation d'au moins un indole substitué, le cas échéant sous forme de son racémate, ses stéréo-isomères purs,

en particulier les énantiomères ou les diastéréo-isomères, ou sous forme de mélanges des stéréo-isomères, en particulier des énantiomères ou des diastéréo-isomères, dans un rapport de mélange quelconque ou à chaque fois sous forme de son acide ou de sa base ou sous forme de son sel, en particulier d'un sel physiologiquement acceptable, ou sous forme de son solvate, en particulier de l'hydrate, selon l'une quelconque des revendications 1 à 13 pour la préparation d'un médicament destiné au traitement ou à la prophylaxie de maladies de neuro-dégénérescence, de préférence de la maladie d'Alzheimer, de Parkinson ou de Huntington, destiné au traitement ou à la prophylaxie d'une attaque, d'une ischémie cérébrale, d'un infarctus cérébral, d'un oedème cérébral, d'états de sous-approvisionnement du système nerveux central, de préférence de l'hypoxie ou de l'anoxie, de l'épilepsie, de la schizophrénie, de psychoses provoquées par un niveau élevé d'acides aminés, de la démence liée au SIDA, de l'encéphalomyélite, du syndrome de Tourette, de l'asphyxie périnatale, du Tinnitus, de la migraine, de réactions inflammatoires et/ou allergiques, de dépressions, de maladies psychiques, de l'incontinence urinaire, du prurit ou de la diarrhée ou destiné à l'anxiolyse ou l'anesthésie.